# EUROPEAN PATENT APPLICATION

(11) **EP 2 602 243 A1**
(43) Date of publication of application: **12.06.2013**
(21) Application number: 11814745.3
(22) Date of filing: 05.08.2011
(51) Int. Cl.: C07C 211/54, C07C 211/57, C07C 211/61, C09K 11/06, H01L 51/50

(54) **MONOAMINE DERIVATIVE AND ORGANIC ELECTROLUMINESCENT ELEMENT USING SAME**

(30) Priority: 20.08.2010 JP 2010185094; 05.08.2010 JP 2010176718
(71) Applicant: Idemitsu Kosan Co., Ltd., Chiyoda-ku Tokyo 100-8321 (JP)
(72) Inventor: ITO Mitsunori, Sodegaura-shi Chiba 299-0293 (JP); OGIWARA Toshinari, Sodegaura-shi Chiba 299-0293 (JP); NISHIMURA Kazuki, Sodegaura-shi Chiba 299-0293 (JP); INOUE Tetsuya, Sodegaura-shi Chiba 299-0293 (JP); HIBINO Kumiko, Sodegaura-shi Chiba 299-0293 (JP)
(74) Representative: Gille Hrabal
(86) International application number: PCT/JP2011/067962
(87) International publication number: WO 2012/018120

(57) **Abstract**

An organic electroluminescence device (1) includes: an anode (3); a cathode (4) being opposed to the anode (3); and an emitting layer (5) being provided between the anode (3) and the cathode (4). The emitting layer (5) includes a host material and a phosphorescent dopant material. The host material includes a monoamine derivative represented by a formula (1A) below. 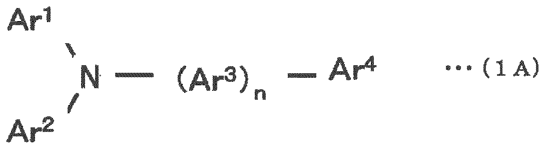 In the formula (1A), each of Ar¹, Ar² and Ar⁴ is a substituted or unsubstituted aryl group or heteroaryl group, Ar³ is a substituted or unsubstituted arylene group or heteroarylene group, n is an integer of 0 to 5, Ar³ may be mutually the same or different when n is 2 or more, and at least one of Ar¹, Ar² and Ar⁴ is a group derived from a fused aromatic hydrocarbon skeleton having 3 or more rings.

## Description

### TECHNICAL FIELD

The present invention relates to a monoamine derivative and an organic electroluminescence device using the same.

### BACKGROUND ART

Organic electroluminescence devices (hereinafter occasionally abbreviated as "organic EL device"), which include organic thin-film layers (in which emitting layers are included) between anodes and cathodes, have been known to emit light using exciton energy generated by a recombination of holes and electrons that have been injected into the emitting layers (see, for instance, Patent Literatures 1 to 5).

Patent Literatures 1 to 5 disclose monoamine derivatives used in organic EL devices. It is disclosed that these monoamine derivatives each include a group derived from a benzene ring, naphthalene ring, phenanthrene ring, dibenzofuran ring or fluorene ring.
The monoamine derivatives disclosed in Patent Literatures 1 and 2 are combined with a fluorescent material, namely TBPe (2,5,8,11-tetra-tert-butylperylene) or 1,2-bis(4-binaphthylaminophenyl)ethylene to be used as a fluorescent host material.
The monoamine derivative disclosed in Patent Literature 3, whish is a fluorescent compound, is used to form an emitting layer in an organic EL device.

There has been known a typical organic EL device in which an emitting layer is provided by one host material and a phosphorescent dopant material. Such an organic EL device is likely to suffer from unbalanced transport of electrons or holes in the emitting layer. In this case, due to the presence of an excessive number of electrons or holes that are irrelevant to recombination, a method of adjusting carrier balance between electrons and holes has been sought to prevent a reduction in luminous efficiency.
For instance, there have been proposed organic EL devices in each of which a first host material having electron transporting capability and a second host material having hole transporting capability are used in an emitting layer to adjust carrier balance (see Patent Literatures 6 to 9).
Patent Literature 6 discloses an organic EL device including an emitting layer that uses CBP (4,4'-bis(carbazole)biphenyl) as the first host material having electron transporting capability and uses TPD (N,N'-bis(3-methylphenyl)-N,N'-diphenylbenzidine, diamine derivative) as the second host material having hole transporting capability. The luminous efficiency of the organic EL device disclosed in Patent Literature 6 can be significantly changed by changing the concentration of TPD (the second host material).
Patent Literature 7 discloses an organic EL device including a fluorescent-emitting layer that uses 2-(4'-tert-butylphenyl)-5-(4"-biphenyl)-1,3,4-oxadiazole as the first host material having electron transporting capability and uses TPD as the second host material having hole transporting capability.
Patent Literatures 8 and 9 each disclose an organic EL device including an emitting layer that uses Alq₃ (tris-(8-hydroxyquinoline)aluminum) as the first host material having electron transporting capability and uses NPD (N,N'-diphenyl-N,N'-bis-alpha-naphthylbenzidine, diamine derivative) as the second host material having hole transporting capability.

### CITATION LIST

### PATENT LITERATURES

Patent Literature 1: JP-T-2008-545729
Patent Literature 2: JP-A-2009-215333
Patent Literature 3: JP-A-2000-12229
Patent Literature 4: JP-A-2008-37755
Patent Literature 5: JP-A-2009-283899
Patent Literature 6: JP-A-2004-296185
Patent Literature 7: JP-A-4-212286
Patent Literature 8: JP-T-2004-515895
Patent Literature 9: JP-A-2004-172068

### SUMMARY OF THE INVENTION

### PROBLEMS TO BE SOLVED BY THE INVENTION

Patent Literatures 1 to 5 disclose monoamine derivatives each including a benzene ring or the like as described above.
These monoamine derivatives may be used as a phosphorescent host material and combined with a phosphorescent material to improve external quantum efficiency and the like.
However, since a phosphorescent host material is required to exhibit a higher triplet energy than the combined phosphorescent material, it is not successful to simply apply the monoamine derivatives disclosed in Patent Literatures 1 to 5 as a phosphorescent host material.
A typical amine derivative is vulnerable to electrons and has a significantly short lifetime, which is shorter than that of a carbazole derivative or the like typically used as a phosphorescent host material (e.g., CBP). Thus, it is difficult to apply the amine derivatives disclosed in Patent Literatures 1 to 5, which are intended to be used as a fluorescent host material, as a phosphorescent host material.
Patent Literature 2 discloses using a non-amine compound or a diamino compound as a phosphorescent host material. Any material used as a phosphorescent host material should ensure not only triplet energy but also charge transporting capability. However, the material disclosed in Patent Literature 2 fails to satisfy all of such requirements, and thus it is difficult to provide an organic EL device that exhibits high efficiency and has a long lifetime.

An organic EL device in which the first host material and the second host material are used in the emitting layer (e.g., the organic EL devices disclosed in Patent Literatures 6 and 7, in each of which TPD is used as the second host material) has luminous efficiency largely dependent on the concentration of TPD. Each of the organic EL devices using NPD disclosed in Patent Literatures 8 and 9 also has luminous efficiency or the like largely dependent on the concentration of NPD in the same manner as the organic EL device disclosed in Patent Literature 6. Thus, the organic EL devices disclosed in Patent Literatures 6 to 9 in which the second host is used are not suitable for mass production because the concentration of the second host material needs to be finely adjusted to a specific level for achieving high luminous efficiency during manufacturing the organic EL devices.

A first object of the invention is to provide a monoamine derivative and an organic EL device using the same, the monoamine derivative contributing to improvement in the luminous efficiency, external quantum efficiency and emission lifetime of the organic EL device.
A second object of the invention is to provide an organic EL device being excellent in luminous efficiency and external quantum efficiency and suitable for mass production even when a first host material and a second host material are used in an emitting layer.

### MEANS FOR SOLVING THE PROBLEMS

As a result of concentrated studies for achieving the above objects, the inventors have found out that a monoamine derivative containing a specific fused aromatic hydrocarbon group contributes to improvement in the luminous efficiency, external quantum efficiency and emission lifetime of an organic EL device.
Furthermore, as a result of concentrated studies for achieving the above objects, the inventors have found out that, when an organic EL device employs a monoamine derivative having a specific skeleton as a second host material, the organic EL device exhibits excellent luminous efficiency and external quantum efficiency and is suitable for mass production.
The invention has been made based on these findings.

[1] According to an aspect of the invention, an organic electroluminescence device includes: an anode; a cathode being opposed to the anode; and an emitting layer being provided between the anode and the cathode, in which the emitting layer includes a host material and a phosphorescent dopant material, and the host material includes a monoamine derivative represented by a formula (1A) below.

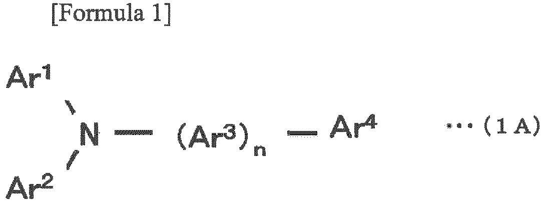

In the formula (1A), each of Ar¹, Ar² and Ar⁴ is a substituted or unsubstituted aryl group or heteroaryl group.
Ar³ is a substituted or unsubstituted arylene group or heteroarylene group.
n is an integer of 0 to 5. When n is 2 or more, Ar³ may be mutually the same or different.
At least one of Ar¹, Ar², Ar³ and Ar⁴ is a group derived from a fused aromatic hydrocarbon skeleton having 3 or more rings.

[2] Preferably, in the above organic electroluminescence device of [1], the fused aromatic hydrocarbon skeleton having 3 or more rings is any one of skeletons represented by formulae (2A) to (5A) below.

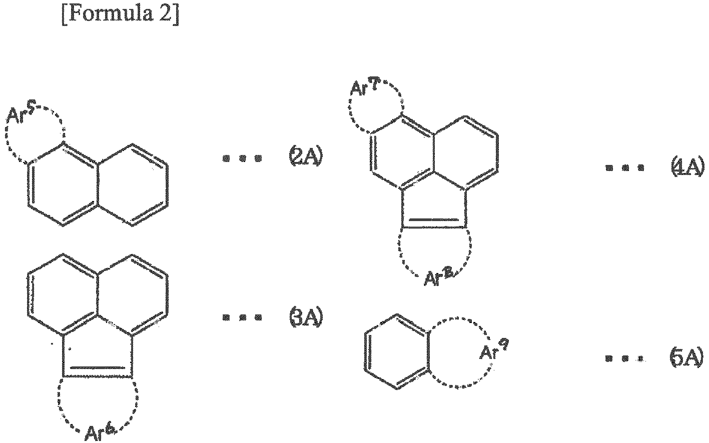

In the formulae (2A) to (5A), each of Ar⁵ to Ar⁹ has a substituted or unsubstituted cyclic structure having 4 to 20 ring carbon atoms and is combined with a ring adjacent thereto to form a fused ring.

[3] Preferably, in the above organic electroluminescence device of [1] or [2], the emitting layer includes: a first host material; a second host material; and a phosphorescent dopant material, and the second host material is the monoamine derivative.

[4] Preferably, in the above organic electroluminescence device of [3], Ar⁴ in the formula (1A) for the second host material is a group derived from the fused aromatic hydrocarbon skeleton having 3 or more rings.

[5] Preferably, in the above organic electroluminescence device of [3], Ar⁴ in the formula (1A) for the second host material is a monovalent group derived from any one of the following skeletons: a phenanthrene skeleton; a benzophenanthrene skeleton; a dibenzophenanthrene skeleton; a chrysene skeleton; a benzochrysene skeleton; a dibenzochrysene skeleton; a fluoranthene skeleton; a benzofluoranthene skeleton; a triphenylene skeleton; a benzotriphenylene skeleton; a dibenzotriphenylene skeleton; a picene skeleton; a benzopicene skeleton; and a dibenzopicene skeleton.

[6] Preferably, in the above organic electroluminescence device of any one of [1] to [5], the phosphorescent dopant material is an ortho-metalated complex of a metal selected from iridium (Ir), osmium (Os) and platinum (Pt).

[7] According to another aspect of the invention, a monoamine derivative is represented by a formula (1) below.

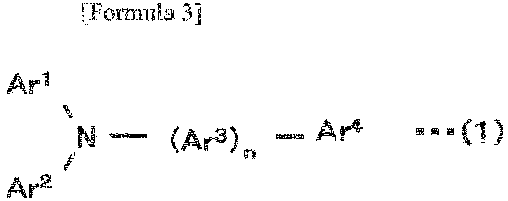

In the formula (1), each of Ar¹, Ar² and Ar⁴ is a substituted or unsubstituted aryl group or heteroaryl group. Ar³ is a substituted or unsubstituted arylene group or heteroarylene group.
n is an integer of 0 to 5.
When n is 0, at least one of Ar¹, Ar² and Ar⁴ is a group derived from a fused aromatic hydrocarbon skeleton having 3 or more rings.
When n is 1, at least one of Ar¹, Ar², Ar³ and Ar⁴ is a group derived from the fused aromatic hydrocarbon skeleton having 3 or more rings.
When n is 2 or more, Ar³ may be mutually the same or different.

[8] Preferably, in the above monoamine derivative of [7], when n is 2 or more in the formula (1), at least one of Ar¹, Ar² and Ar⁴ is a group derived from the fused aromatic hydrocarbon skeleton having 3 or more rings.

[9] Preferably, in the above monoamine derivative of [7] or [8], the fused aromatic hydrocarbon skeleton having 3 or more rings is any one of skeletons represented by formulae (2) to (5) below.

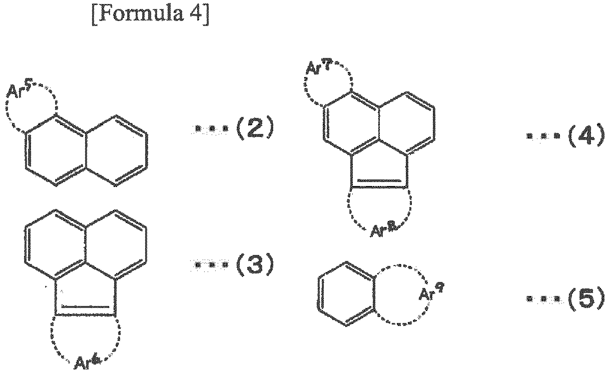

In the formulae (2) to (5), each of Ar⁵, Ar⁶, Ar⁷, Ar⁸ and Ar⁹ has a substituted or unsubstituted cyclic structure having 4 to 20 ring carbon atoms and is combined with a ring adjacent thereto to form a fused ring.

[10] Preferably, in the above monoamine derivative of [9], Ar⁴ in the formula (1) is a monovalent group derived from any one of the skeletons represented by the formulae (2) to (5).

[11] Preferably, in the above monoamine derivative of [10], Ar⁴ in the formula (1) is a monovalent group derived from any one of the following skeletons: a phenanthrene skeleton; a benzophenanthrene skeleton; a dibenzophenanthrene skeleton; a chrysene skeleton; a benzochrysene skeleton; a dibenzochrysene skeleton; a fluoranthene skeleton; a benzofluoranthene skeleton; a triphenylene skeleton; a benzotriphenylene skeleton; a dibenzotriphenylene skeleton; a picene skeleton; a benzopicene skeleton; and a dibenzopicene skeleton.

[12] Preferably, in the above monoamine derivative of [7], when n is 0, the monoamine derivative is represented by a formula (6) below, and at least one of Ar¹, Ar² and Ar⁴ is a fused aromatic hydrocarbon group selected from the group consisting of: a phenanthrenyl group; a benzophenanthrenyl group; a dibenzophenanthrenyl group; a benzochrysenyl group; a dibenzochrysenyl group; a fluoranthenyl group; a benzofluoranthenyl group; a triphenylenyl group; a benzotriphenylenyl group; a dibenzotriphenylenyl group; a picenyl group; a benzopicenyl group; a dibenzopicenyl group; a phenalenyl group; an acenaphthenyl group; and a diazaphenanthrenyl group.

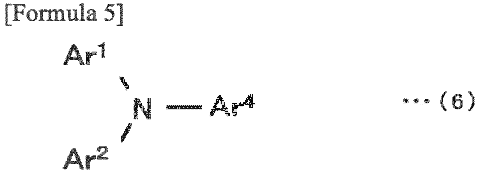

[13] Preferably, in the above monoamine derivative of [7], when n is 1, the monoamine derivative is represented by a formula (7) below, Ar⁴ is a fused aromatic hydrocarbon group selected from the group consisting of: a phenanthrenyl group; a benzophenanthrenyl group; a dibenzophenanthrenyl group; a benzochrysenyl group; a dibenzochrysenyl group; a fluoranthenyl group; a benzofluoranthenyl group; a triphenylenyl group; a benzotriphenylenyl group; a dibenzotriphenylenyl group; a picenyl group; a benzopicenyl group; and a dibenzopicenyl group. When Ar³ is a phenylene group or 1,4-naphthylene group, Ar⁴ is not a phenanthrenyl group bonded to Ar³ at 2,3,9-positions.

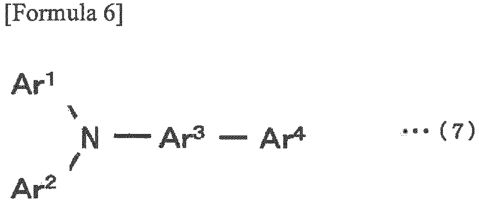

[14] Preferably, in the above monoamine derivative of [7], when n is 2 or more, Ar⁴ is an aromatic hydrocarbon group or a fused aromatic hydrocarbon group selected from the group consisting of: a phenyl group; a naphthyl group; a phenanthrenyl group; a benzophenanthrenyl group; a dibenzophenanthrenyl group; a benzochrysenyl group; a dibenzochrysenyl group; a fluoranthenyl group; a benzofluoranthenyl group; a triphenylenyl group; a benzotriphenylenyl group; a dibenzotriphenylenyl group; a picenyl group; a benzopicenyl group; a dibenzopicenyl group; a phenalenyl group; and a diazaphenanthrenyl group. When each of Ar¹ and Ar² is a phenyl group, (Ar³)n-Ar⁴ is not structured as represented by a formula (8) below.

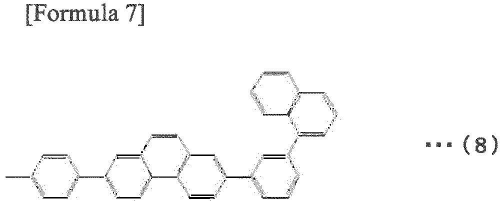

[15] Preferably, in the above monoamine derivative of [12], each of Ar¹ and Ar² is a phenyl group or a naphthyl group, and Ar⁴ is any one of a benzochrysenyl group, triphenylenyl group and phenanthrenyl group.

[16] Preferably, in the above monoamine derivative of [13], each of Ar¹ and Ar² is a phenyl group or a naphthyl group, Ar³ is a phenylene group or a naphthylene group, and Ar⁴ is any one of a benzochrysenyl group, triphenylenyl group and phenanthrenyl group.

[17] Preferably, in the above monoamine derivative of [14], each of Ar¹ and Ar² is a phenyl group or a naphthyl group, Ar³ is a phenylene group or a naphthylene group, and Ar⁴ is any one of a benzochrysenyl group, triphenylenyl group and phenanthrenyl group.

[18] Preferably, the above monoamine derivative of any one of [7] to [17] is usable as a phosphorescent host material in an organic electroluminescence device.

[19] According to still another aspect of the invention, an organic electroluminescence device includes: a cathode; an anode; and a single-layered or multilayered organic thin-film layer provided between the cathode and the anode, in which at least one layer of the organic thin-film layer includes the monoamine derivative of any one of [7] to [17].

[20] Preferably, in the above organic electroluminescence device of [19], the organic thin-film layer including the monoamine derivative further includes at least one phosphorescent material.

[21] Preferably, in the above organic electroluminescence device of [19], the at least one layer of the organic thin-film layer is an emitting layer, and at least one layer of the emitting layer includes the monoamine derivative and at least one phosphorescent material.

[22] Preferably, in the above organic electroluminescence device of [21], the emitting layer includes: a first host material; a second host material; and a phosphorescent dopant material, the second host material includes the monoamine derivative, and the phosphorescent dopant material is the at least one phosphorescent material.

[23] Preferably, in the above organic electroluminescence device of [22], the first host material is a fused aromatic compound represented by any one of formulae (11) and (12) below.

   Ar^{a}-Ar^{b}-Ar^{c} (11)

   Ar^{d}-Ar^{e}-Ar^{f}-Ar^{g} (12)

   In the formulae (11) and (12), Ar^{a}, Ar^{b}, Ar^{c}, Ar^{d}, Ar^{e}, Ar^{f} and Ar^{g} each independently represents a group derived from any one of the following skeletons: a substituted or unsubstituted benzene skeleton; a substituted or unsubstituted naphthalene skeleton; and a substituted or unsubstituted fused aromatic hydrocarbon skeleton having 3 or more rings.

[24] Preferably, in the above organic electroluminescence device of [23], the fused aromatic hydrocarbon skeleton having 3 or more rings for the first host material is any one of skeletons represented by formulae (13) to (16) below.

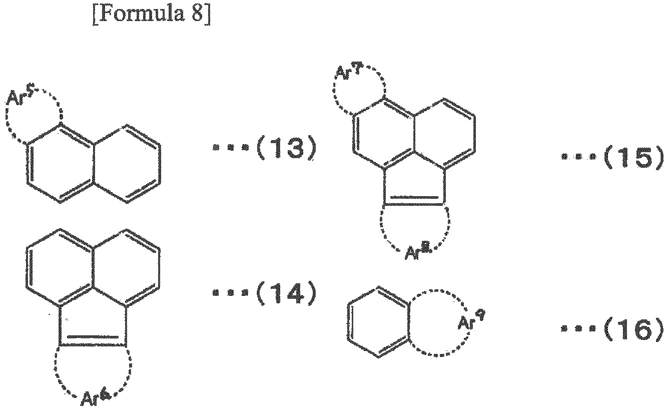

In the formulae (13) to (16), each of Ar⁵, Ar⁶, Ar⁷, Ar⁸ and Ar⁹ has a substituted or unsubstituted cyclic structure having 4 to 20 ring carbon atoms and is combined with a ring adjacent thereto to form a fused ring.

[25] Preferably, in the above organic electroluminescence device of [24], the fused aromatic hydrocarbon skeleton having 3 or more rings for the first host material is any of skeletons: a phenanthrene skeleton; a benzophenanthrene skeleton; a dibenzophenanthrene skeleton; a chrysene skeleton; a benzochrysene skeleton; a dibenzochrysene skeleton; a fluoranthene skeleton; a benzofluoranthene skeleton; a triphenylene skeleton; a benzotriphenylene skeleton; a dibenzotriphenylene skeleton; a picene skeleton; a benzopicene skeleton; and a dibenzopicene skeleton.

[26] Preferably, in the above organic electroluminescence device of any one of [20] to [25], the phosphorescent material includes a metal complex, and the metal complex includes: a metal atom selected from the group consisting of Ir, Pt, Os, Au, Re and Ru; and a ligand.

[27] Preferably, in the above organic electroluminescence device of [26], the ligand has an ortho-metal bond with the metal atom of the metal complex.

[28] Preferably, in the above organic electroluminescence device of any one of [20] to [27], a maximum emission wavelength of the at least one phosphorescent material contained in the emitting layer is in a range of 520 nm to 720 nm.

[29] According to still another aspect of the invention, a monoamine derivative is represented by a formula (1B) below.

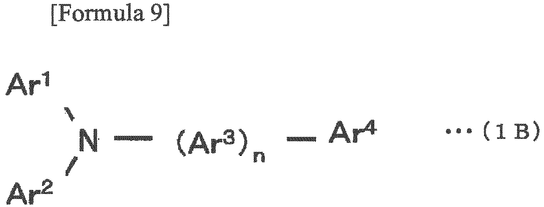

In the formula (1B), each of Ar¹, Ar² and Ar⁴ is a substituted or unsubstituted aryl group or heteroaryl group.
Ar³ is a substituted or unsubstituted arylene group or heteroarylene group.
n is an integer of 0 to 5. When n is 2 or more, Ar³ may be mutually the same or different.
When n is 1, Ar³ is a substituted or unsubstituted arylene group having 12 to 20 ring carbon atoms or heteroarylene group having 5 to 20 ring atoms.
At least one of Ar¹, Ar² and Ar⁴ is a group derived from a fused aromatic hydrocarbon skeleton having 3 or more rings.

According to the above aspect(s) of the invention, a monoamine derivative having a specific structure can improve the luminous efficiency, external quantum efficiency and emission lifetime of an organic EL device.
According to the above aspect(s) of the invention, an organic EL device is excellent in luminous efficiency and external quantum efficiency and suitable for mass production even when a first host material and a second host material are used in an emitting layer.

### BRIEF DESCRIPTION OF DRAWINGS

Fig. 1 schematically shows an arrangement of an organic EL device according to a first exemplary embodiment of the invention.
Fig. 2 schematically shows an arrangement of an organic EL device according to a fourth exemplary embodiment of the invention.

### DESCRIPTION OF EMBODIMENTS

### Monoamine Derivative

A monoamine derivative according to the invention is represented by the following formula (1).

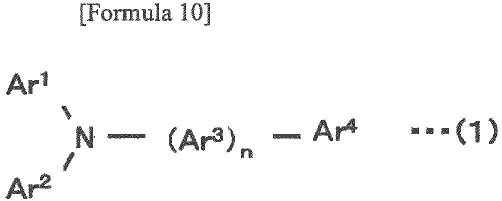

In the formula (1), each of Ar¹, Ar² and Ar⁴ is a substituted or unsubstituted aryl group or heteroaryl group.
The aryl group has 6 to 50 carbon atoms forming a ring (hereinafter referred to as ring carbon atoms) (preferably 6 to 30 ring carbon atoms, more preferably 6 to 20 ring carbon atoms). Examples of the aryl group are a phenyl group, naphthyl group, phenanthrenyl group, benzophenanthrenyl group, dibenzophenanthrenyl group, benzochrysenyl group, dibenzochrysenyl group, fluoranthenyl group, benzofluoranthenyl group, triphenylenyl group, benzotriphenylenyl group, dibenzotriphenylenyl group, picenyl group, benzopicenyl group, dibenzopicenyl group, phenalenyl group, acenaphthenyl group, and diazaphenanthrenyl group. Among the above, a phenyl group or naphthyl group is preferable.
The heteroaryl group has 5 to 50 atoms forming a ring (hereinafter referred to as ring atoms) (preferably 6 to 30 ring atoms, more preferably 6 to 20 ring atoms). Examples of the heteroaryl group are a pyrimidyl group and diazaphenanthrenyl group.

In the formula (1), Ar³ is a substituted or unsubstituted arylene group or heteroarylene group.
The arylene group has 6 to 50 ring carbon atoms (preferably 6 to 30 ring carbon atoms, more preferably 6 to 20 ring carbon atoms). Examples of the arylene group are a phenylene group, naphthylene group, phenanthrenylene group, naphthacenylene group, pyrenylene group, biphenylene group, terphenylenylene group, benzophenanthrenylene group, dibenzophenanthrenylene group, benzochrysenylene group, dibenzochrysenylene group, fluoranthenylene group, benzofluoranthenylene group, triphenylenylene group, benzotriphenylenylene group, dibenzotriphenylenylene group, picenylene group, benzopicenylene group, and dibenzopicenylene group. Among the above, a phenylene group or naphthylene group is preferable.
The heteroaryl group has 5 to 50 ring atoms (preferably 6 to 30 ring atoms, more preferably 6 to 20 ring atoms). Examples of the heteroaryl group are a pyridylene group, pyrimidylene group, dibenzofuranylene group, and dibenzothiophenylene group.
Ar³ is preferably a phenylene group or naphthylene group.

In the formula (1), n is an integer of 0 to 5, preferably 1 to 4, more preferably 1 to 3. In other words, in the formula (1), it is preferable that a triarylamine skeleton and a fused aromatic hydrocarbon skeleton having 3 or more rings separately exist.
In the formula (1), when n is 0, at least one of Ar¹, Ar² and Ar⁴ is a group derived from the fused aromatic hydrocarbon skeleton having 3 or more rings.
In the formula (1), when n is 1, at least one of Ar¹, Ar², Ar³ and Ar⁴ is a group derived from the fused aromatic hydrocarbon skeleton having 3 or more rings.
In the formula (1), when n is 2 or more, Ar³ may be mutually the same or different. When n is 2 or more, at least one of Ar¹, Ar² and Ar⁴ is preferably a group derived from the fused aromatic hydrocarbon skeleton having 3 or more rings.

The fused aromatic hydrocarbon skeleton having 3 or more rings is preferably any one of skeletons represented by the following formulae (2) to (5).

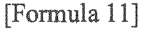

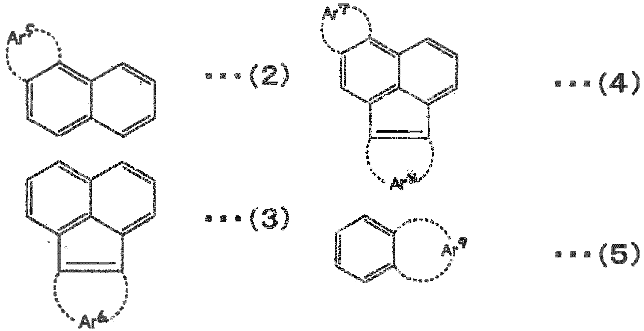

In the formulae (2) to (5), each of Ar⁵, Ar⁶, Ar⁷, Ar⁸ and Ar⁹ has a substituted or unsubstituted cyclic structure having 4 to 20 ring carbon atoms and is combined with a ring adjacent thereto to form a fused ring.

Ar⁴ in the formula (1) is further preferably a monovalent group derived from any one of the skeletons represented by the formulae (2) to (5).

Ar⁴ in the formula (1) is preferably a monovalent group derived from any one of a phenanthrene skeleton, benzophenanthrene skeleton, dibenzophenanthrene skeleton, chrysene skeleton, benzochrysene skeleton, dibenzochrysene skeleton, fluoranthene skeleton, benzofluoranthene skeleton, triphenylene skeleton, benzotriphenylene skeleton, dibenzotriphenylene skeleton, picene skeleton, benzopicene skeleton, and dibenzopicene skeleton.
Among the above, Ar⁴ is preferably a monovalent group derived from any one of a benzochrysene skeleton, triphenylene skeleton and phenanthrene skeleton.

In the formula (1), when n is 0, the monoamine derivative is represented by the above formula (6). At least one of Ar¹, Ar² and Ar⁴ is a fused aromatic hydrocarbon group selected from a phenanthrenyl group, benzophenanthrenyl group, dibenzophenanthrenyl group, benzochrysenyl group, dibenzochrysenyl group, fluoranthenyl group, benzofluoranthenyl group, triphenylenyl group, benzotriphenylenyl group, dibenzotriphenylenyl group, picenyl group, benzopicenyl group, dibenzopicenyl group, phenalenyl group, acenaphthenyl group, and diazaphenanthrenyl group. Among the above, a benzochrysenyl group, triphenylenyl group, or phenanthrenyl group is preferable. The fused aromatic hydrocarbon group does not have a substituent.
In the monoamine derivative represented by the formula (6) according to the invention, Ar¹ and Ar² each are preferably a phenyl group or naphthyl group, and Ar⁴ is preferably a benzochrysenyl group, triphenylenyl group, or phenanthrenyl group.
When at least one of Ar¹, Ar² and Ar⁴ is the above specific ring, it is assumed that using the monoamine derivative as an organic-EL-device material results in an improvement in charge transporting capability, and thus in an improvement in the luminous efficiency, external quantum efficiency and emission lifetime of an organic EL device. Additionally, triplet energy can be ensured, so that the monoamine derivative is usable as a phosphorescent host material.

In the formula (1), when n is 1, the monoamine derivative is represented by the above formula (7). Ar⁴ is a fused aromatic hydrocarbon group selected from a phenanthrenyl group, benzophenanthrenyl group, dibenzophenanthrenyl group, benzochrysenyl group, dibenzochrysenyl group, fluoranthenyl group, benzofluoranthenyl group, triphenylenyl group, benzotriphenylenyl group, dibenzotriphenylenyl group, picenyl group, benzopicenyl group, and dibenzopicenyl group. When Ar³ is a phenylene group or 1,4-naphthylene group, Ar⁴ is not a phenanthrenyl group bonded to Ar³ at 2,3,9-positions. Among the above, a benzochrysenyl group, triphenylenyl group, or phenanthrenyl group is preferable. The fused aromatic hydrocarbon group does not have a substituent.
In the monoamine derivative represented by the formula (7) according to the invention, it is preferable that each of Ar¹ and Ar² is a phenyl group or naphthyl group, Ar³ is a phenylene group or naphthylene group, and Ar⁴ is a benzochrysenyl group, triphenylenyl group, or phenanthrenyl group.
When Ar⁴ is the above specific ring, it is assumed that using the monoamine derivative as an organic-EL-device material results in an improvement in charge transporting capability, and thus in an improvement in the luminous efficiency, external quantum efficiency and emission lifetime of an organic EL device. Additionally, triplet energy can be ensured, so that the monoamine derivative is usable as a phosphorescent host material.

In the formula (1), when n is 2 or more, each of Ar¹ and Ar² is preferably a phenyl group or naphthyl group, and Ar³ is preferably a phenylene group or naphthylene group.
In the formula (1), when n is 2 or more, Ar⁴ is an aromatic hydrocarbon group or fused aromatic hydrocarbon group selected from a phenyl group, naphthyl group, phenanthrenyl group, benzophenanthrenyl group, dibenzophenanthrenyl group, benzochrysenyl group, dibenzochrysenyl group, fluoranthenyl group, benzofluoranthenyl group, triphenylenyl group, benzotriphenylenyl group, dibenzotriphenylenyl group, picenyl group, benzopicenyl group, dibenzopicenyl group, phenalenyl group, and diazaphenanthrenyl group. Among the above, a benzochrysenyl group, triphenylenyl group, or phenanthrenyl group is preferable. When each of Ar¹ and Ar² is a phenyl group, (Ar³)ₙ-Ar⁴ is not structured as represented by the above formula (8).

In the monoamine derivative represented by the formula (1), when n is 2 or more, it is preferable that each of Ar¹ and Ar² is a phenyl group or naphthyl group, Ar³ is a phenylene group or naphthylene group, and Ar⁴ is a benzochrysenyl group, triphenylenyl group, or phenanthrenyl group.
When Ar⁴ is the above specific ring, it is assumed that using the monoamine derivative as an organic-EL-device material results in an improvement in charge transporting capability, and thus in an improvement in the luminous efficiency, external quantum efficiency and emission lifetime of an organic EL device. Additionally, triplet energy can be ensured, so that the monoamine derivative is usable as a phosphorescent host material.

When the monoamine derivative is used as a phosphorescent host material, it is assumed that the luminous efficiency, external quantum efficiency and lifetime of an organic EL device are significantly improved as compared with a typical material having a diamine structure because the monoamine derivative has a monoamine structure and the specific ring represented by Ar⁴ in one molecule as in Example(s).

When Ar¹ to Ar⁴ have substituent(s), the substituent(s) is preferably an alkyl group having 1 to 20 carbon atoms, haloalkyl group having 1 to 20 carbon atoms, cycloalkyl group having 3 to 18 carbon atoms, aryl group having 6 to 30 ring carbon atoms, silyl group having 3 to 20 carbon atoms, cyano group, or halogen atom.
Examples of the alkyl group are a methyl group, ethyl group, propyl group, isopropyl group, n-butyl group, 1-methylpropyl group and 1-propylbutyl group.
Examples of the aryl group are the same as those for Ar¹ in the monoamine derivative.
The haloalkyl group is exemplified by a 2,2,2-trifluoroethyl group.
Examples of the cycloalkyl group are a cyclopropyl group, cyclobutyl group, cyclopentyl group, cyclohexyl group and cyclooctyl group.
Examples of the silyl group are a trimethylsilyl group and triethylsilyl group.
Examples of the halogen atom are fluorine, chlorine, bromine, and iodine.
When the monoamine derivative does not have a substituent, it means that a hydrogen atom is substituted. The hydrogen atom of the monoamine derivative includes light hydrogen and deuterium.
"Carbon atoms forming a ring (ring carbon atoms)" herein mean carbon atoms forming a saturated ring, unsaturated ring, or aromatic ring. "Atoms forming a ring (ring atoms)" mean carbon atoms and hetero atoms forming a ring (including a saturated ring, unsaturated ring, and aromatic ring).

When n is 0 in the formula (1) according to the invention, the monoamine derivative is represented by the above formula (6). Examples of the monoamine derivative are shown below. However, the invention is not limited to these exemplary compounds.

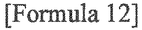

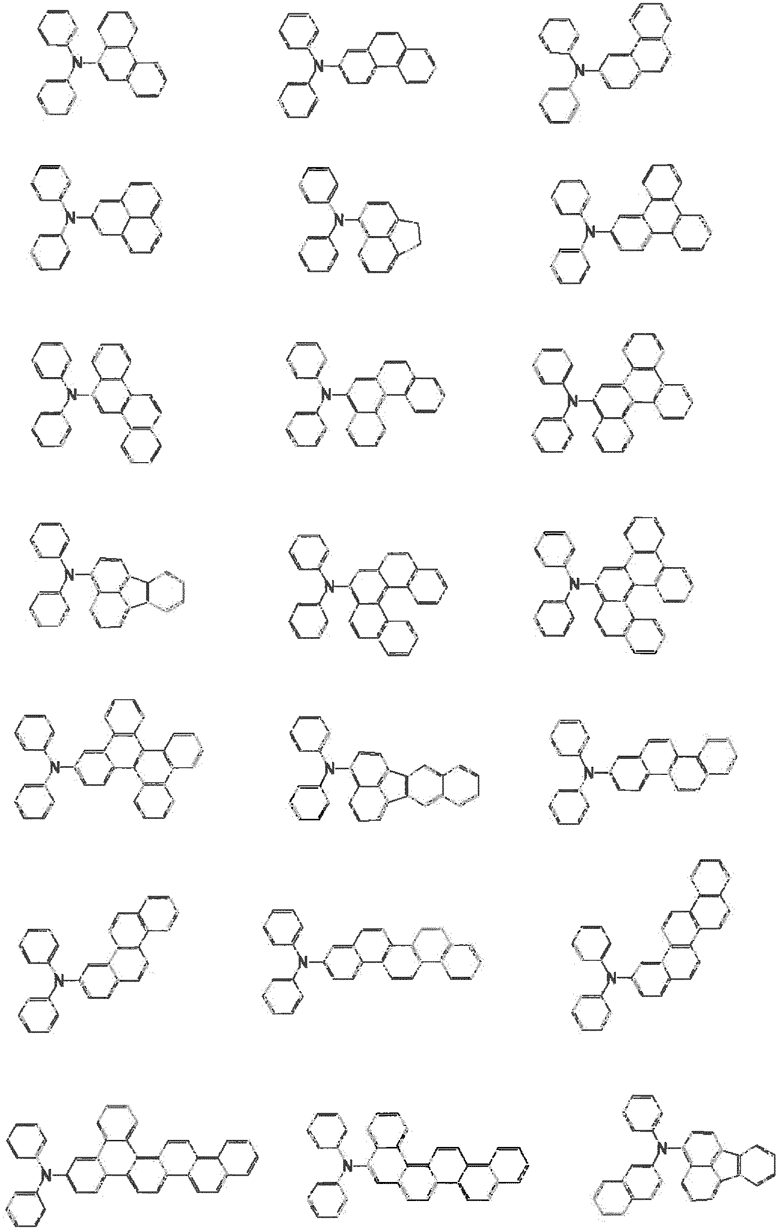

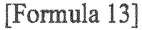

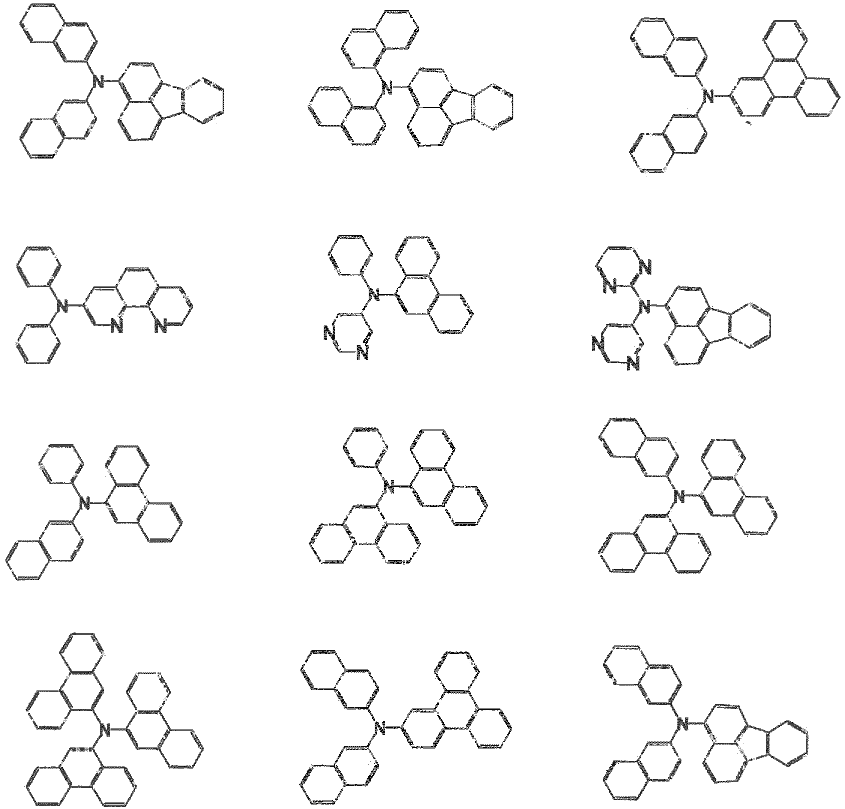

When n is 1 in the formula (1) according to the invention, the monoamine derivative is represented by the above formula (7). Examples of the monoamine derivative are shown below. However, the invention is not limited to these exemplary compounds.

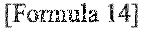

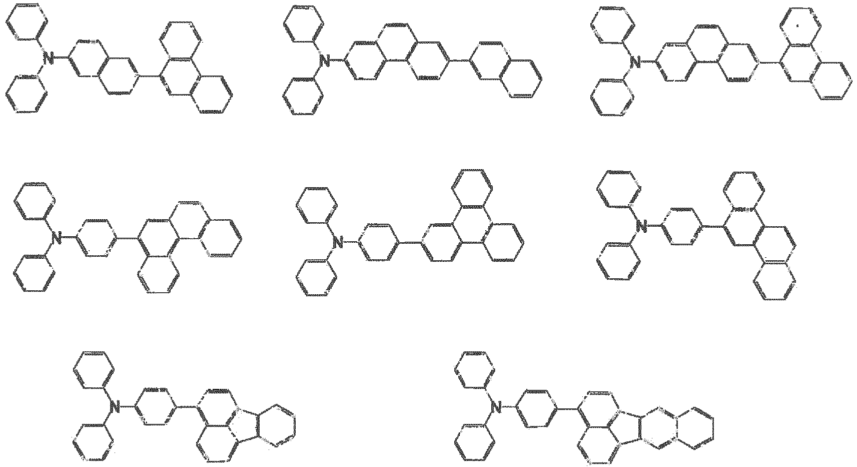

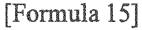

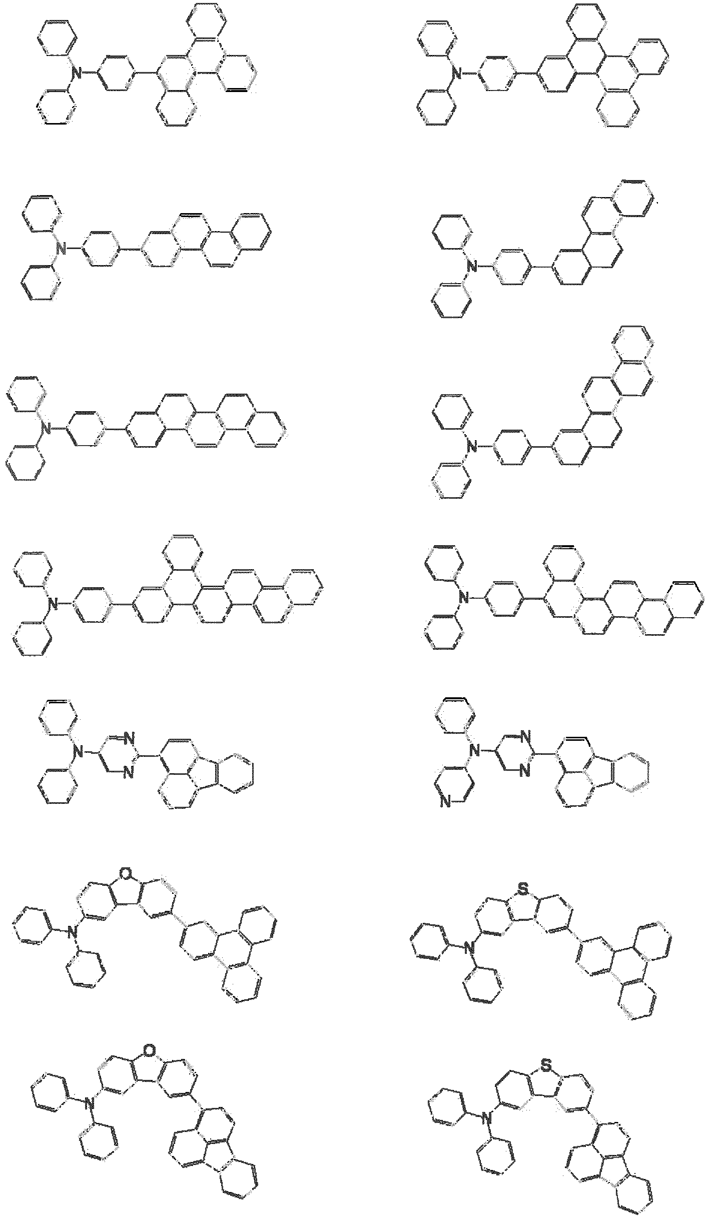

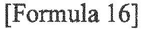

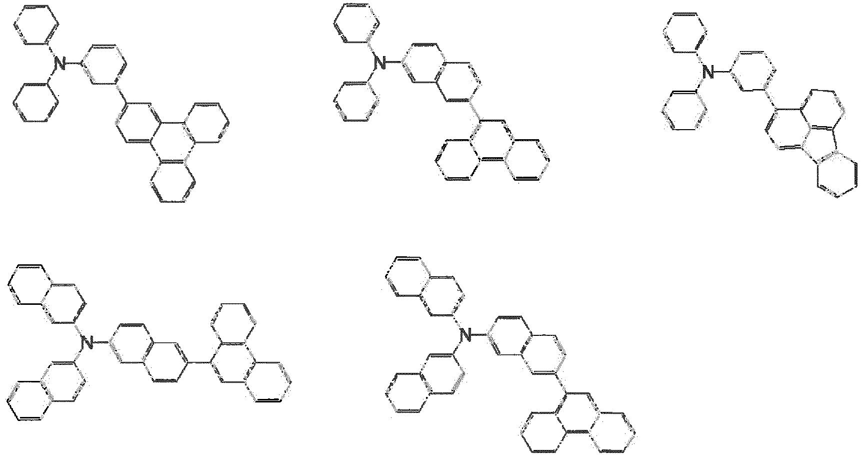

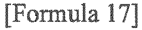

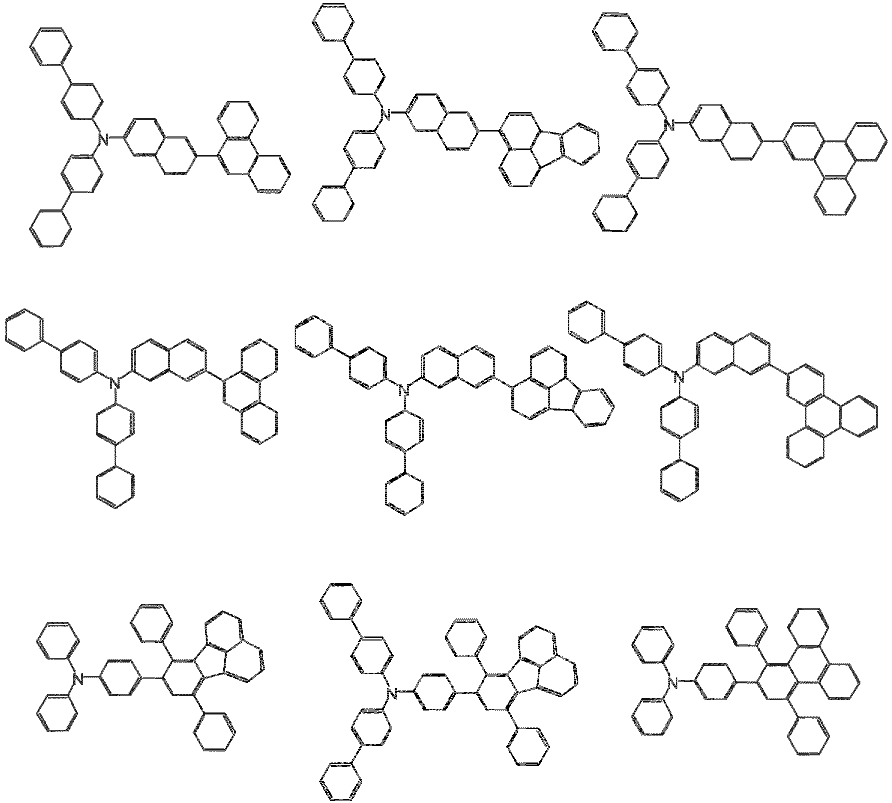

When n is 2 or more in the formula (1) according to the invention, the monoamine derivative is exemplified as follows. However, the invention is not limited to these exemplary compounds.

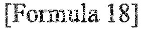

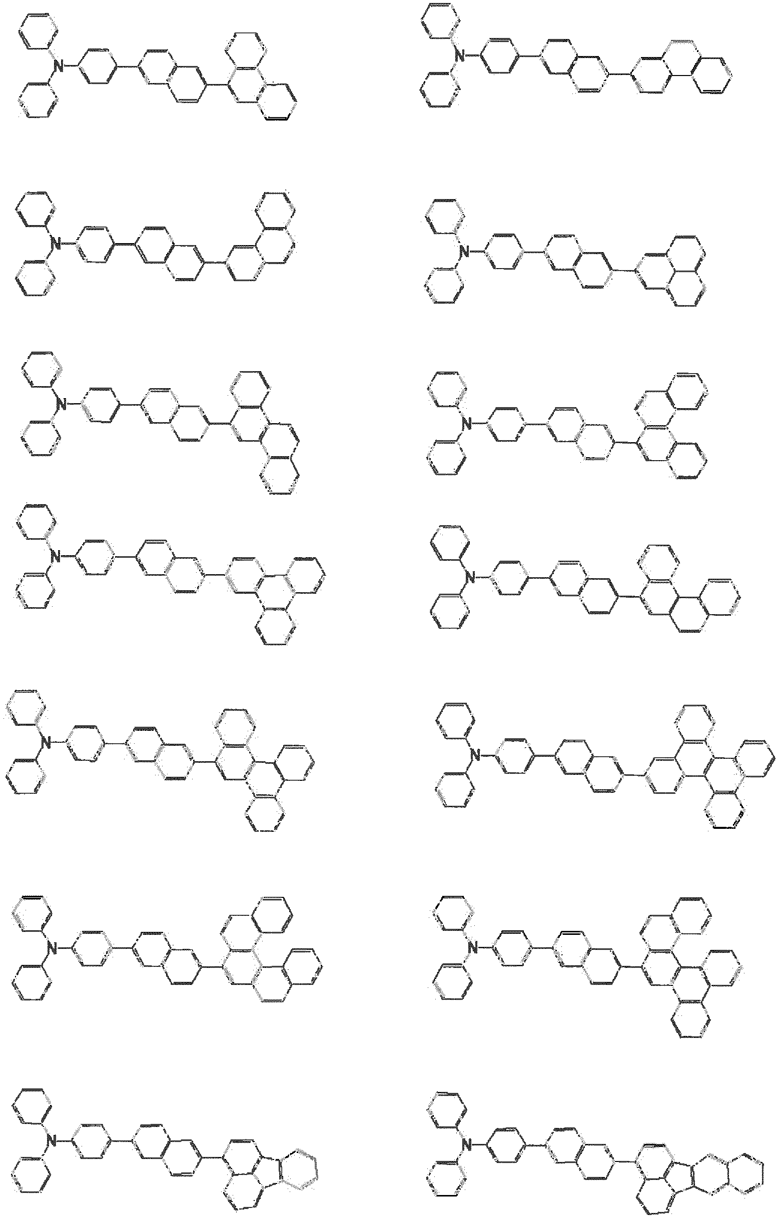

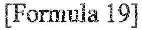

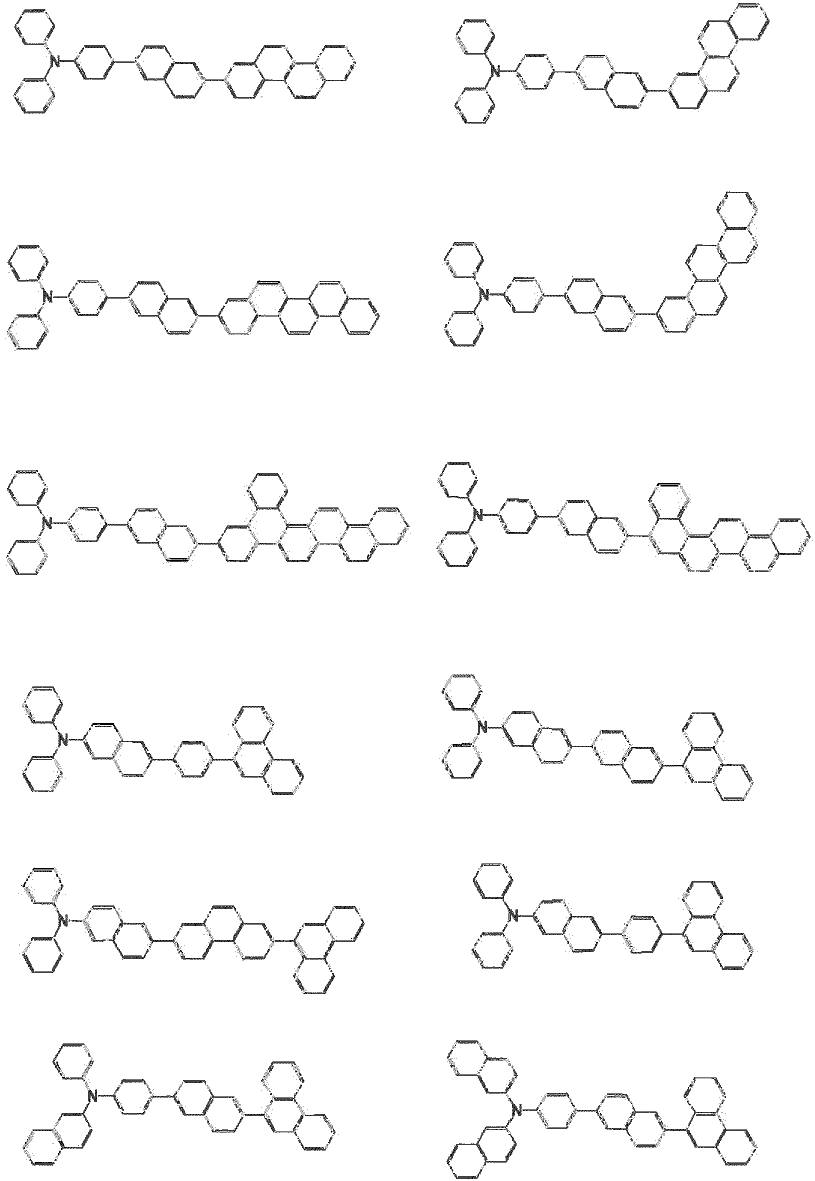

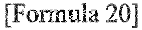

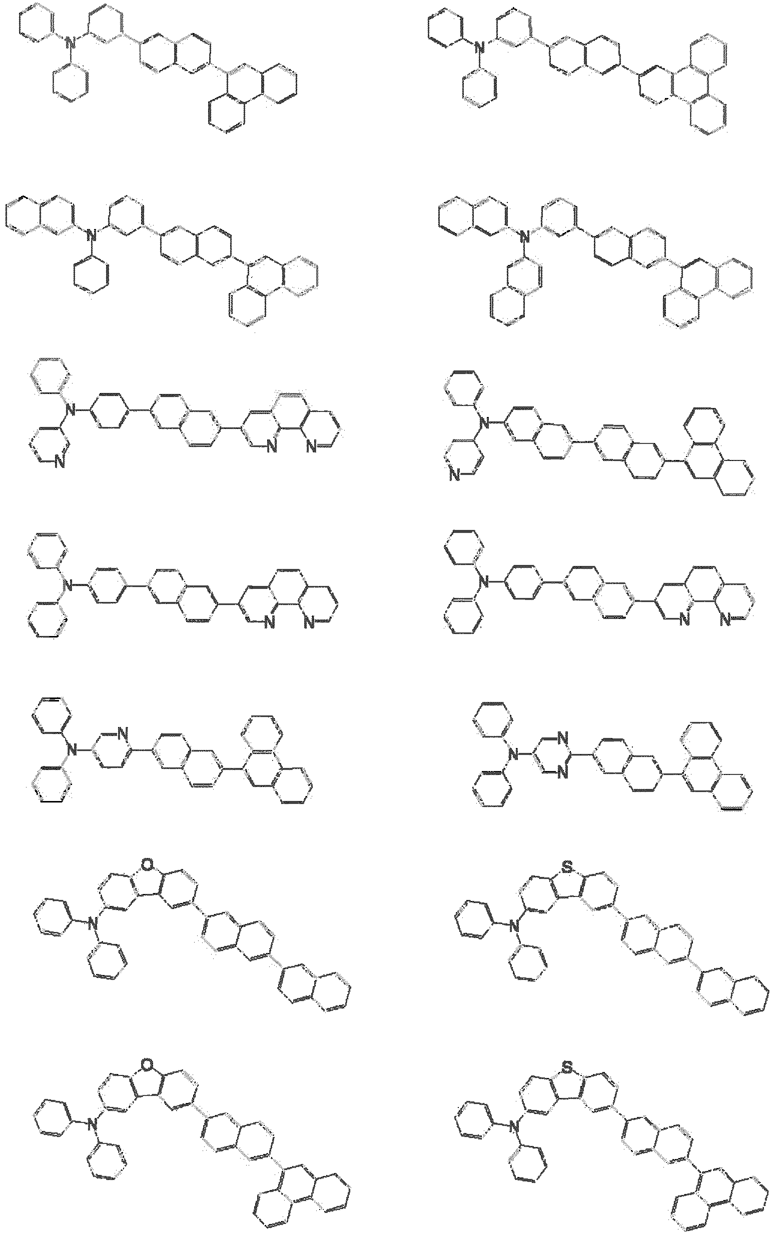

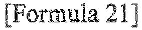

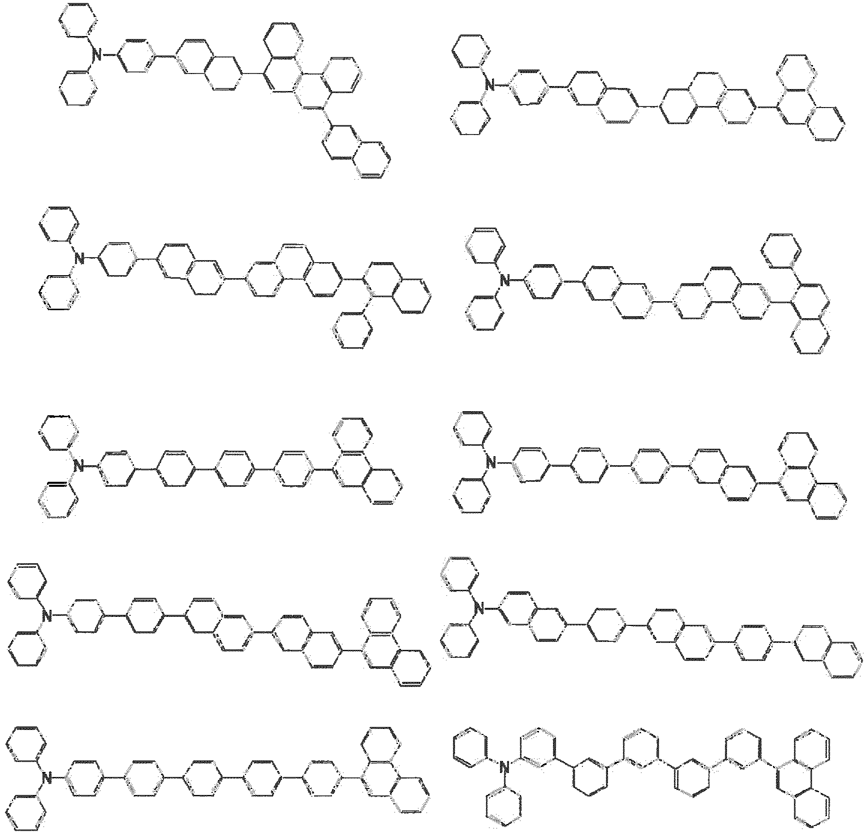

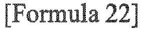

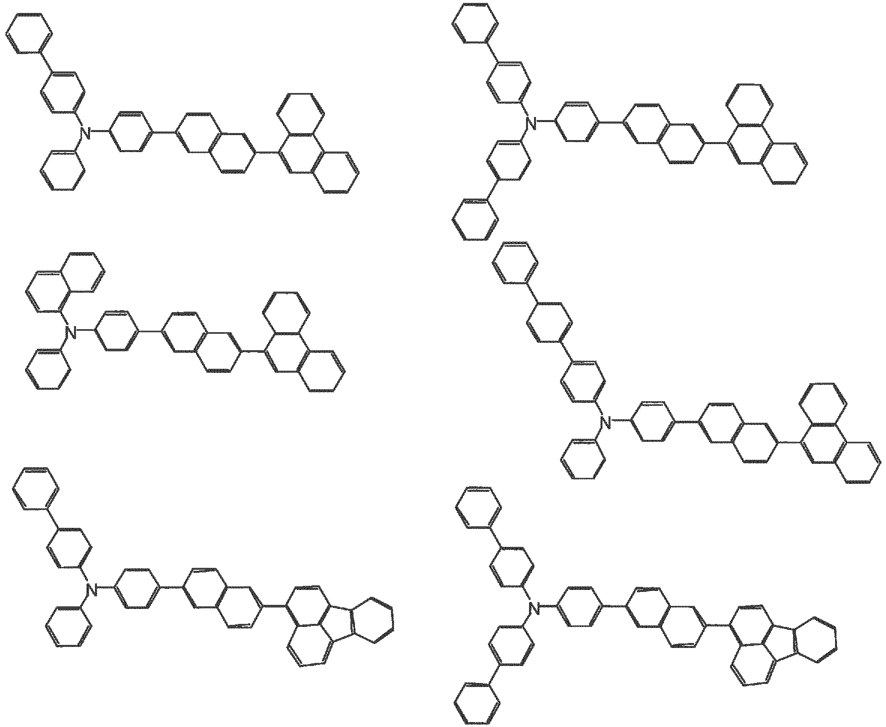

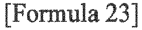

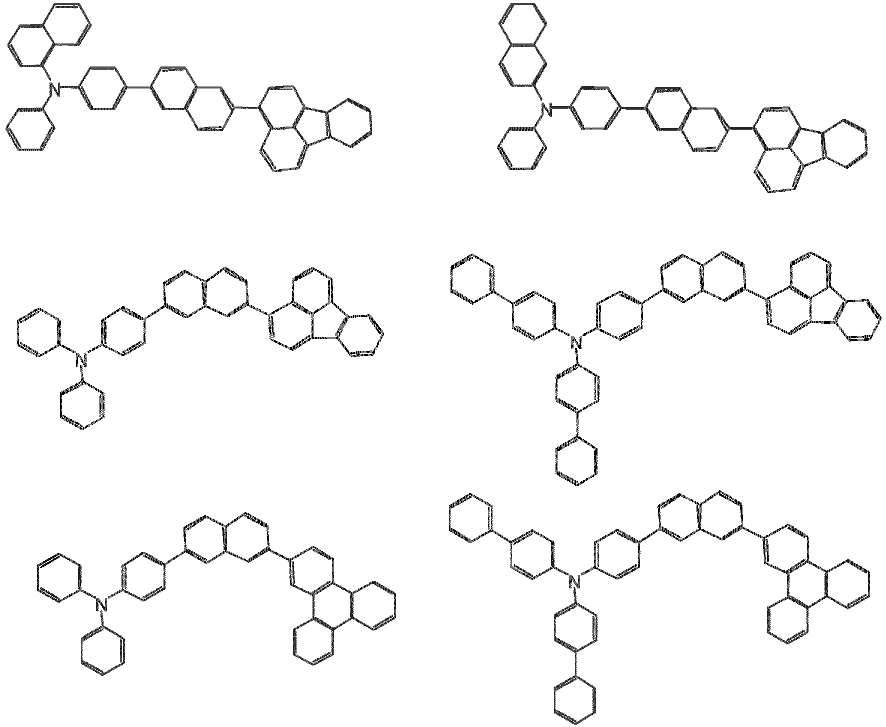

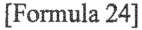

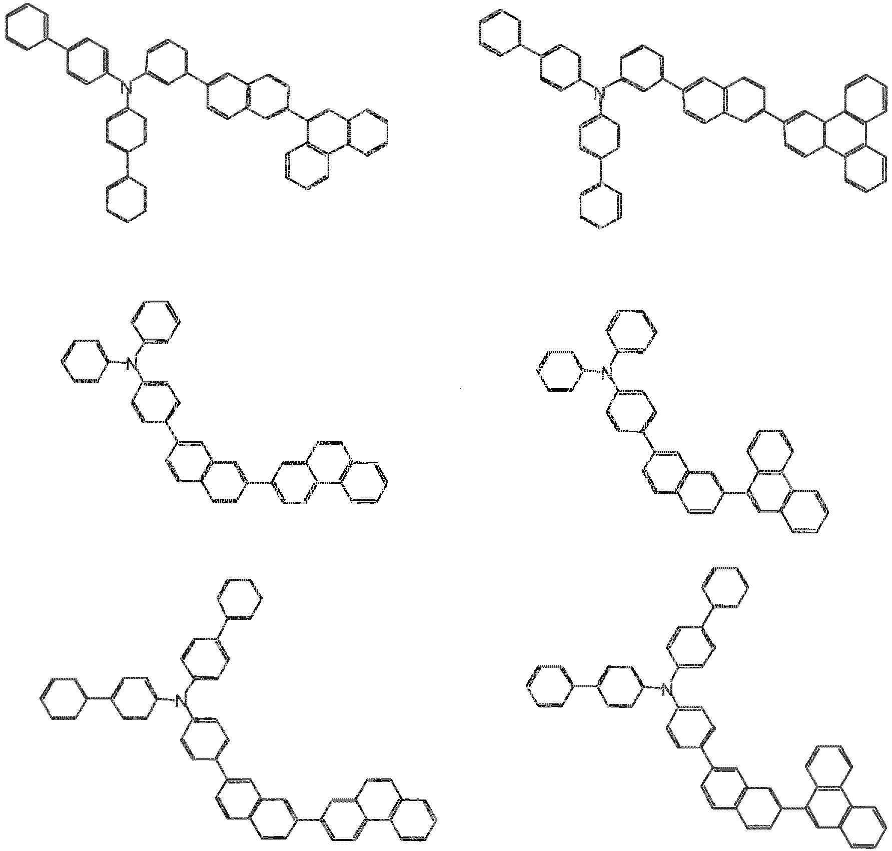

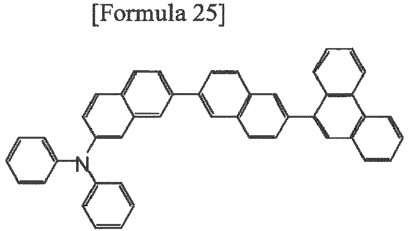

A method of manufacturing the monoamine derivative according to the invention is not particularly limited. The monoamine derivative can be manufactured in a known method. For instance, a coupling reaction between an amine derivative and an aromatic halogenated compound may be usable as a method. Such a coupling reaction is exemplified by a method using a copper catalyst described in Tetrahedron 40 (1984), pp. 435 to 1456 or a method using a palladium catalyst described in The Journal of American Chemical Society 123 (2001), pp. 7727 to 7729.

### Organic EL Device

Next, an organic EL device according to the invention will be described below. First Exemplary Embodiment

An organic EL device according to this exemplary embodiment has an arrangement including at least one emitting layer. Exemplary arrangements are as given below.
(1) anode / emitting layer / electron injecting·transporting layer / cathode
(2) anode / hole injecting layer / emitting layer / electron injecting.transporting layer / cathode
(3) anode / hole injecting layer / hole transporting layer / emitting layer /electron injecting.transporting layer / cathode
(4) anode / hole injecting·transporting layer / emitting layer / electron transporting layer / cathode
Among the above, the exemplary arrangement (4) is preferable, but the invention is not limited thereto.

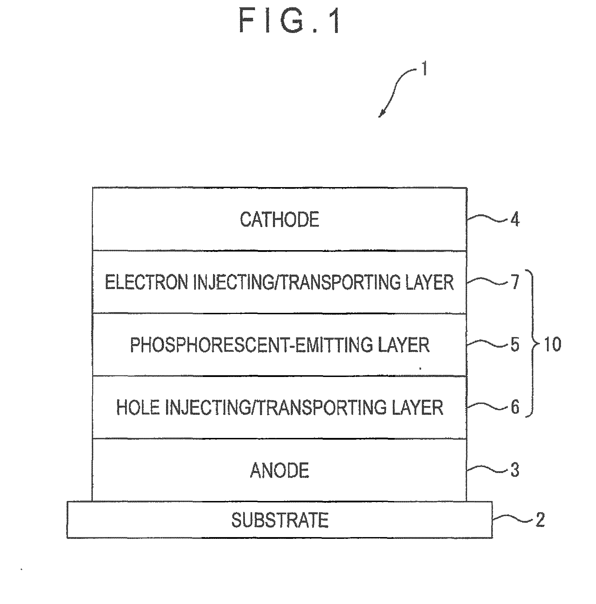

As shown in Fig. 1, an organic EL device 1 includes a transparent substrate 2, an anode 3, a cathode 4 and a multi-layered organic thin-film layer 10 provided between the anode 3 and the cathode 4.
The organic thin-film layer 10 includes a hole injecting/transporting layer 6, an emitting layer 5 and an electron injecting/transporting layer 7 arranged in this sequence between the anode 3 and the cathode 4.
The emitting layer 5 is interposed between the hole injecting/transporting layer 6 and the electron injecting/transporting layer 7, and emits phosphorescent light.
The organic EL device 1 may further include an electron blocking layer provided to the emitting layer 5 near the anode 3 and a hole blocking layer provided to the emitting layer 5 near the cathode 4. With this arrangement, electrons and holes can be trapped in the emitting layer 5, thereby enhancing probability of exciton generation in the emitting layer 5.

### Light-Transmissive Substrate

The organic EL device according to this exemplary embodiment is formed on a light-transmissive substrate. The light-transmissive substrate, which supports the organic EL device, is preferably a smoothly-shaped substrate that transmits 50% or more of light in a visible region of 400 nm to 700 nm. The light-transmissive substrate is exemplarily a glass plate or a polymer plate.
For the glass plate, materials such as soda-lime glass, barium/strontium-containing glass, lead glass, aluminosilicate glass, borosilicate glass, barium borosilicate glass and quartz can be used.
For the polymer plate, materials such as polycarbonate resins, acryl resins, polyethylene terephthalate resins, polyether sulfide resins and polysulfone resins can be used.

### Anode and Cathode

The anode of the organic EL device serves for injecting holes into the hole injecting/transporting layer or the emitting layer. It is advantageous that the anode has a work function of 4.5 eV or more.
Examples of a material for the anode are alloys of indium-tin oxide (ITO), tin oxide (NESA), indium zinc oxide, gold, silver, platinum and copper.
The anode may be made by forming a thin film from these electrode materials through a method such as vapor deposition and sputtering.
The anode preferably transmits more than 10% of the light in the visible region so that light from the emitting layer is emitted through the anode. Sheet resistance of the anode is preferably several hundreds Ω/ sq. or lower. Although depending on the material of the anode, the thickness of the anode is typically in a range of 10 nm to 1 µm, and preferably in a range of 10 nm to 200 nm.

The cathode serves for injecting electrons into the electron injecting/transporting layer or the emitting layer, and thus is preferably formed of a material with a smaller work function.
Although a material for the cathode is subject to no specific limitation, examples of the material are indium, aluminum, magnesium, alloy of magnesium and indium, alloy of magnesium and aluminum, alloy of aluminum and lithium, alloy of aluminum, scandium and lithium, and alloy of magnesium and silver.
Like the anode, the cathode may be made by forming a thin film from the above materials through a method such as vapor deposition and sputtering. Light may be emitted through the cathode of the organic EL device.

### Emitting Layer

The emitting layer has the following functions (1) to (3).
(1) injecting function: a function for accepting, when an electrical field is applied, the holes injected by the anode or the hole injecting/transporting layer, or the electrons injected by the cathode or the electron injecting/transporting layer
(2) transporting function: a function for transporting injected electric charges (the electrons and the holes) by the force of the electrical field
(3) emitting function: a function for providing a condition for a recombination of the electrons and the holes to emit light.
Injectability of the holes may differ from that of the electrons and transporting capabilities of the hole and the electrons (represented by mobilities of the holes and the electrons) may differ from each other.

The emitting layer preferably contains at least one of the above monoamine derivatives according to the invention as a phosphorescent host material.
Using the monoamine derivative according to the invention as a phosphorescent host material improves the luminous efficiency, external quantum efficiency and emission lifetime of an organic EL device.
The monoamine derivative is preferably contained in the emitting layer, further preferably contained at a ratio of 1 mass% to 99 mass%, particularly preferably contained at a ratio of 3 mass% to 95 mass%, the most preferably contained at a ratio of 5 mass% to 90 mass%.
The thickness of the emitting layer is preferably in a range of 5 nm to 50nm, more preferably 7 nm to 50 nm, the most preferably 10 nm to 50 nm. When the thickness is 5 nm or more, the emitting layer can be easily formed. When the thickness is 50 nm or less, chromaticity can be easily controlled to suppress an increase in driving voltage.
It should be noted that a phosphorescent host material, which is used to form an organic thin-film layer containing a phosphorescent material, exhibits a larger triplet energy than the phosphorescent material.

### Phosphorescent Material

When the emitting layer of the organic EL device contains the monoamine derivative as a phosphorescent host material, the emitting layer preferably contains at least one phosphorescent material. According to the exemplary embodiment, the phosphorescent material is a phosphorescent dopant material when being used in the emitting layer.
The phosphorescent material contained in the emitting layer generates phosphorescent emission, and preferably contains a metal complex. The metal complex preferably includes: a metal atom selected from Ir (iridium), Pt (platinum), Os (osmium), Au (gold), Re (rhenium) and Ru (ruthenium); and a ligand. Particularly, the ligand preferably has an ortho-metal bond with the metal atom.
The phosphorescent material preferably contains a metal selected from Ir, Os and Pt because such a metal exhibits a high phosphorescence quantum yield to further enhance the external quantum efficiency of the organic EL device. The phosphorescent material is further preferably a metal complex such as an Ir complex, Os complex and Pt complex. Among the above, an Ir complex and Pt complex are more preferable, and an ortho-metalated Ir complex, which has an ortho-metal bond with the metal atom, is the most preferable.
An example of the ortho-metalated Ir complex is Ir(piq)₃.
At least one phosphorescent material preferably emits light having a maximum emission wavelength of 520 nm to 720 nm, more preferably 570 nm to 720 nm.
In other words, the phosphorescent material preferably enables phosphorescent emission of green to red.
Examples of the phosphorescent material are shown below, but the invention is not limited thereto.

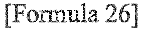

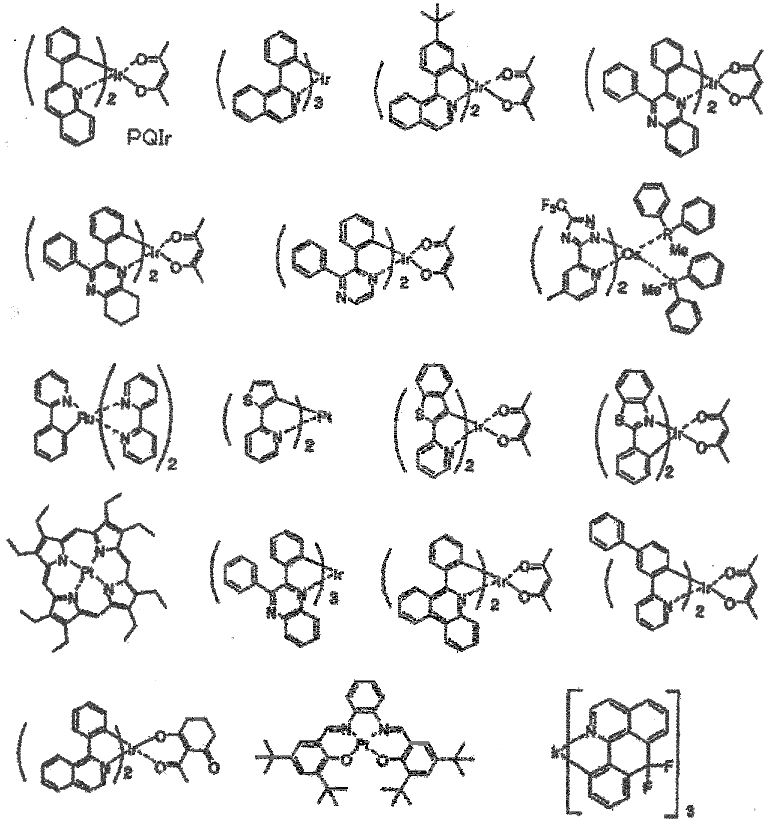

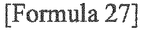

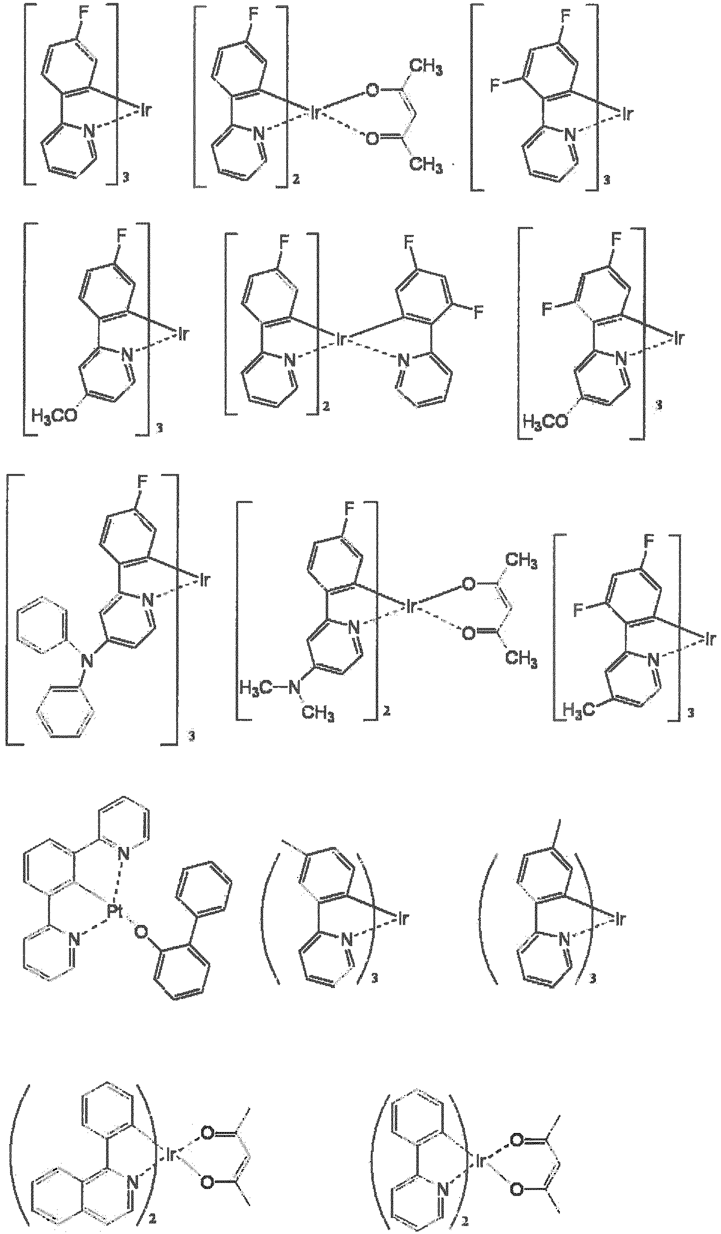

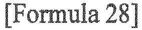

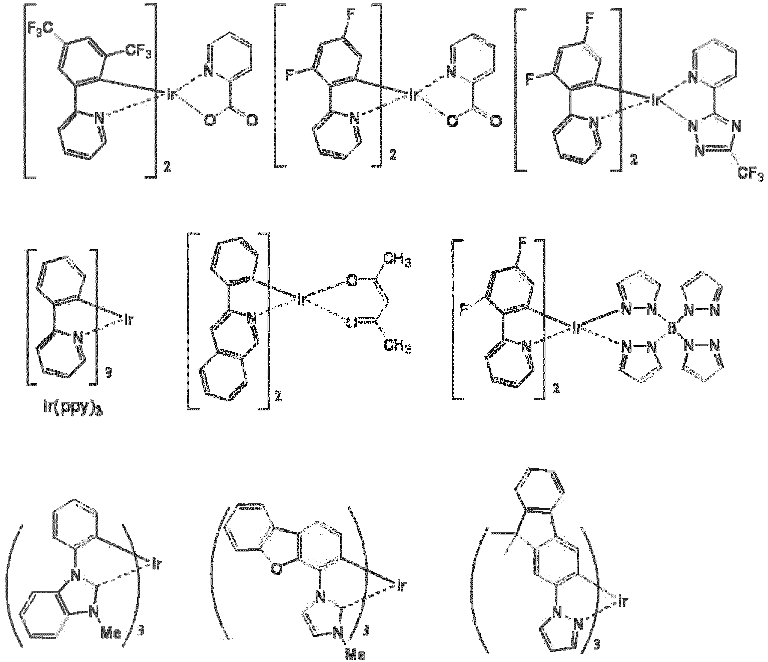

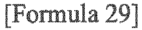

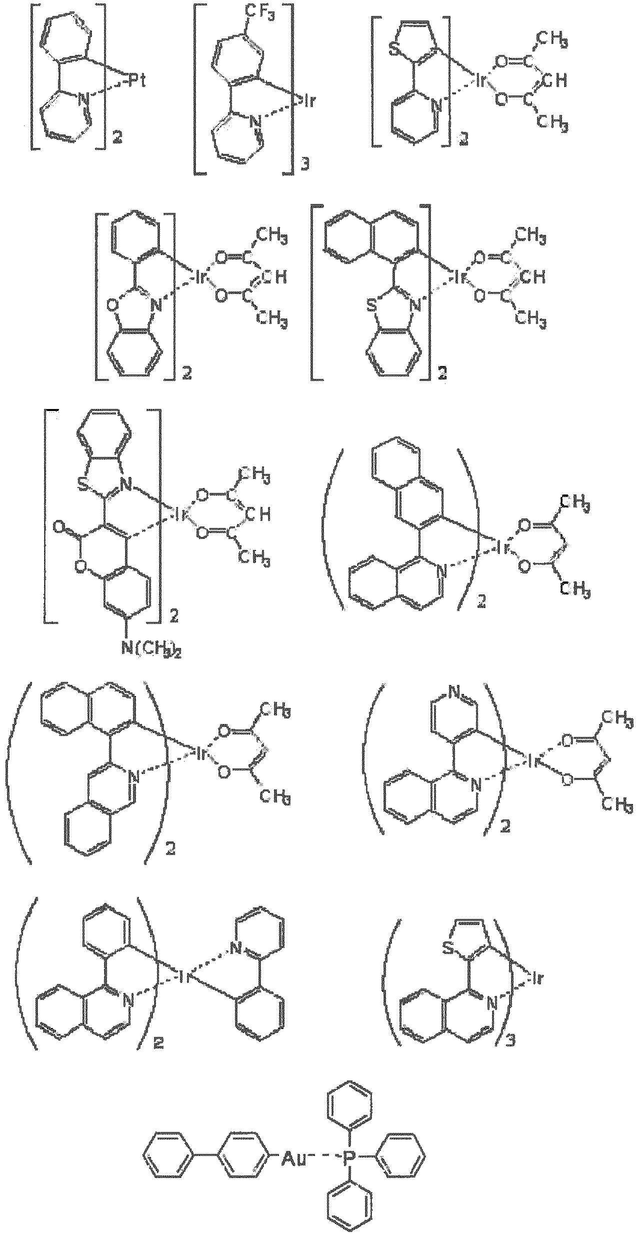

When the organic EL device includes two or more emitting layers, each of the two or more emitting layers may contain the monoamine derivative and the phosphorescent material.
The monoamine derivative and the phosphorescent material according to the invention may be contained in any other organic thin-film layer than the emitting layer. For instance, each of the monoamine derivative and the phosphorescent material according to the invention may be contained in the hole injecting/transporting layer, the electron injecting/transporting layer, the electron blocking layer, the hole blocking layer or the like.

### Hole Injecting/Transporting Layer

The hole injecting/transporting layer, which helps injection of holes into the emitting layer, has a high hole mobility. The hole injecting/transporting layer is provided by at least one of a hole injecting layer and a hole transporting layer. Alternatively, the hole injecting/transporting layer may be a single layer having hole injecting capability and hole transporting capability.
A material for the hole injecting/transporting layer is preferably, for instance, an aromatic amine derivative represented by the following formula (I).

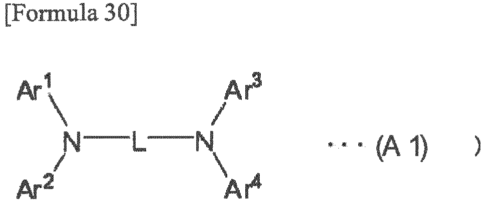

In the formula (I), Ar¹ to Ar⁴ each represents an aryl group having 6 to 50 carbon atoms forming an aromatic ring or a heteroaryl group having 3 to 50 atoms forming an aromatic ring.
Such an aryl group or heteroaryl group is the same as one represented by Ar¹ in the above monoamine derivative.
L is a bonding group, examples of which are an arylene group having 6 to 50 carbon atoms forming an aromatic ring and a heteroarylene group having 5 to 50 carbon atoms forming an aromatic ring. Examples of the arylene group are a phenylene group, biphenyl group, naphthylene group and anthracenylene group. Examples of the heteroarylene group are a pyrrolylene group and pyrazinylene group.
L may be a divalent group formed by bonding two or more arylene groups or heteroarylene groups together via a direct bond, ether bond, thioether bond, alkylene group having 1 to 20 carbon atoms, alkenylene group having 2 to 20 carbon atoms or amino group.
Such a compound represented by the formula (I), an example of which is given in US2009/0009067A1, paragraph [0287], is preferably the following compound.

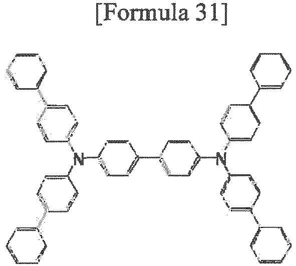

As a material for the hole injecting/transporting layer, an aromatic amine represented by the following (II) is also preferable.

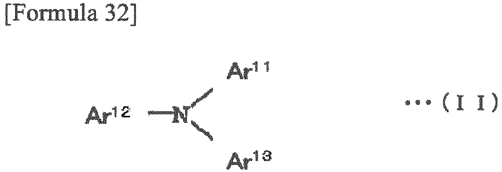

In the above (II), Ar¹¹ to Ar¹³ each represents the same as one represented by Ar¹ of the above (I). An example of the compound represented by the formula (II) is given in US2009/0009067A1, paragraph [0289].
In the above (I) and (II), when Ar¹ to Ar⁴ and Ar¹¹ to Ar¹³ have substituents, examples of the substituents are the same as those of the substituent for Ar¹ of the above monoamine derivative.

### Electron Injecting/Transporting Layer

The electron injecting/transporting layer, which helps injection of electrons into the emitting layer, has a high electron mobility. The electron injecting/transporting layer is provided by at least one of an electron injecting layer and electron transporting layer. Alternatively, the electron injecting/transporting layer may be a single layer having electron injecting capability and electron transporting capability.
A material having electron transporting capability for forming the electron injecting/transporting layer is preferably an aromatic heterocyclic compound having one or more hetero atoms in the molecule, particularly preferably a nitrogen-containing ring derivative. The nitrogen-containing ring derivative is preferably an aromatic or fused aromatic nitrogen-containing ring derivative having a nitrogen-containing six-membered or five-membered ring skeleton.

Examples of such a material are 8-hydroxyquinoline, 8-hydroxyquinoline derivative and oxadiazole derivative.
The 8-hydroxyquinoline derivative may be a metal complex, an example of which is a metal chelate oxynoid compound containing a chelate of oxine (8-quinolinol or 8-hydroxyquinoline). An example of the metal chelate oxynoid compound is a tris(8-quinolinol)aluminum.
Additionally, the nitrogen-containing ring derivative is also preferably a compound represented by the following formula (10).

[Formula 33] **HAr-L³¹-Ar³¹-Ar³²** **(10)**

In the formula (10), HAr is a monovalent nitrogen-containing heterocyclic group having 3 to 40 carbon atoms forming an aromatic ring.
HAr has a structure exemplarily selected from the following group.

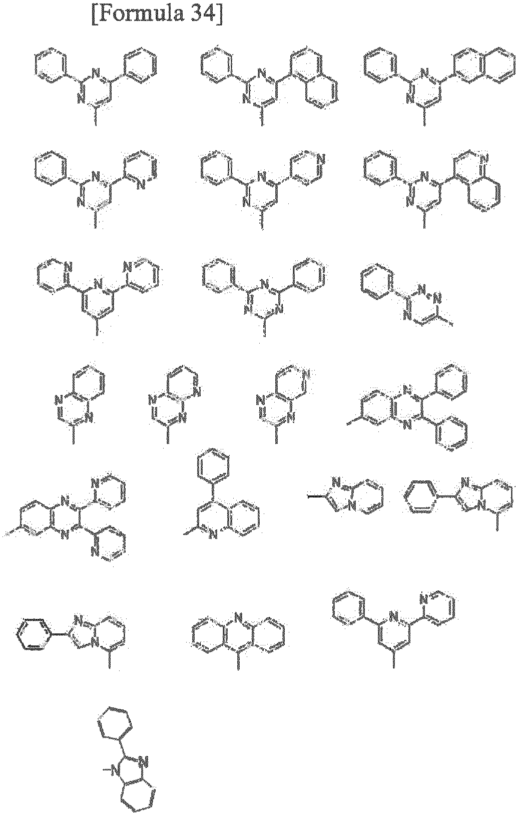

In the formula (10), L³¹ is a single bond, an arylene group having 6 to 50 carbon atoms forming an aromatic ring, or a heteroarylene group having 3 to 50 carbon atoms. L³¹ has a structure exemplarily selected from the following group.

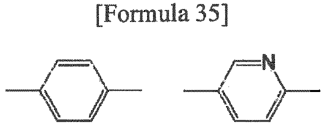

In the formula (10), Ar³¹ is an arylene group having 6 to 50 carbon atoms forming an aromatic ring. Ar³¹ has a structure exemplarily selected from anthracenylene groups of the following formulae (10A).

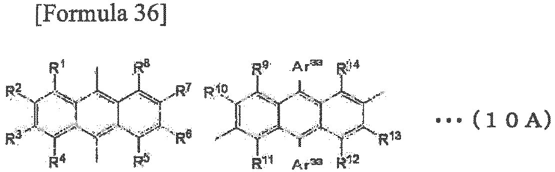

In the formulae (10A), R¹ to R¹⁴ each represents a hydrogen atom, halogen atom, alkyl group having 1 to 20 carbon atoms, alkoxy group having 1 to 20 carbon atoms, aryloxy group having 6 to 50 carbon atoms forming an aromatic ring, aryl group having 6 to 50 carbon atoms forming an aromatic ring, or heteroaryl group having 3 to 50 carbon atoms forming an aromatic ring.
Examples of the halogen atom and the alkyl group having 1 to 20 carbon atoms are the same as those of the substituent for Ar¹ of the above amine derivative.
Examples of the alkoxy group having 1 to 20 carbon atoms are a methoxy group, ethoxy group, isopropoxy group, n-butoxy group, 1-methylpropoxy group and 1-propylbutoxy group.
Examples of the aryloxy group having 6 to 50 carbon atoms forming an aromatic ring are a phenoxy group and naphthoxy group.
Examples of the aryl group having 6 to 50 carbon atoms forming an aromatic ring or the heteroaryl group having 3 to 50 carbon atoms are the same as those of Ar¹ in the formula (I) representing the material for the above hole injecting/transporting layer.
When each of the aryloxy group, aryl group and heteroaryl group has a substituent, examples of the substituent are the same as those of the substituent for Ar¹ of the above monoamine derivative.

In the formulae (10A), Ar³³ is an aryl group having 6 to 50 carbon atoms forming an aromatic ring or a heteroaryl group having 3 to 50 carbon atoms forming an aromatic ring.
The aryl group or heteroaryl group represented by Ar³³ is the same as one represented by R¹ in the formulae (10A).

In the formula (10), Ar³² is an aryl group having 6 to 50 carbon atoms forming an aromatic ring, or a heteroaryl group having 3 to 50 carbon atoms. Ar³² has a structure exemplarily selected from the following group.

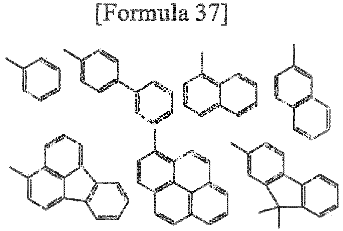

When each of HAr, L³¹, Ar³¹, Ar³² and Ar³³ has a substituent, examples of the substituent are the same as those of the substituent for Ar¹ in the above monoamine derivative.
Such a compound represented by the formula (10), an example of which is given in US2009/0009067A1, paragraphs [0209] to [0261], is preferably the following compound.

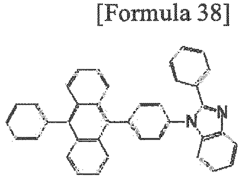

As the nitrogen-containing derivative, compounds disclosed in JP-A-9-3448 and JP-A-2000-173774 are also favorably usable.
The material for the electron injecting/transporting layer may be a polymer compound containing a group derived from the above nitrogen-containing ring derivative.

The electron injecting/transporting layer may contain the monoamine derivative according to the invention as the main component.
It should be noted that "as the main component" means that the monoamine derivative is contained in the electron injecting/transporting layer at a content of 50 mass% or more.
Although the thickness of each layer other than the emitting layer is not particularly limited, the thickness is generally preferably in a range from several nanometers to 1 µm, since an extremely small thickness is likely to cause defects such as a pin hole while an extremely large thickness requires a high voltage to be applied and lowers efficiency.

In addition to the nitrogen-containing ring derivative, an insulator or a semiconductor (an inorganic compound) is also employable as the material for the electron injecting/transporting layer. When the electron injecting/transporting layer is formed of the insulator or the semiconductor, pixel defects such as a dark spot can be reduced and leakage of current can be effectively prevented, thereby improving electron injecting capability and electron transporting capability.
As the insulator, it is preferable to use at least one metal compound selected from the group consisting of an alkali metal chalcogenide, an alkaline earth metal chalcogenide, a halogenide of alkali metal and a halogenide of alkaline earth metal.
Specifically, preferable examples of the alkali metal chalcogenide are Li₂O, K₂O, Na₂S, Na₂Se and Na₂O, while preferable examples of the alkaline earth metal chalcogenide are CaO, BaO, SrO, BeO, BaS and CaSe. Preferable examples of the halogenide of the alkali metal are LiF, NaF, KF, LiCl, KCl and NaCl. Preferable examples of the halogenide of the alkaline earth metal are fluorides such as CaF₂, BaF₂, SrF₂, MgF₂ and BeF₂, and halogenides other than fluorides.
Examples of the semiconductor are one of or a combination of two or more of an oxide, a nitride or an oxidized nitride containing at least one element selected from Ba, Ca, Sr, Yb, Al, Ga, In, Li, Na, Cd, Mg, Si, Ta, Sb and Zn.
When the electron injecting/transporting layer contains such an insulator or a semiconductor, the thickness thereof is preferably in a range of approximately 0.1 nm to 15 nm.

A method of forming each layer of the organic EL device according to the invention is not particularly limited and any known method is usable.
Each layer may be formed in a method such as vacuum deposition and molecular beam epitaxy (MBE method), or a method using a solution such as dipping, spin coating, bar coating, roll coating and LB method.
When being formed by vacuum deposition, each layer can be formed as a thin film. Each thin film may be formed by sequentially accumulating molecules of a material. Such a thin film is exemplarily a thin film formed by depositing a material in gas phase, or a thin film formed by solidifying a material in a solution state or in liquid phase.
Generally, such a thin film can be distinguished from a thin film formed by the LB method (molecular accumulation film) by differences in aggregation structures and higher order structures and functional differences arising therefrom.
For forming each layer by spin coating, a method disclosed in JP-A-57-51781 is usable. Specifically, each layer can be formed using a solution prepared by dissolving a binder (e.g. a resin) and a material in a solvent.

### Second Exemplary Embodiment

An organic EL device according to this exemplary embodiment has a tandem device structure having at least two emitting layers or units each including an emitting layer.
In such an organic EL device, for instance, a charge generating layer (also referred to as CGL) may be interposed between the two units and an electron transporting zone is provided for each unit.
Examples of such a tandem device structure are given below.
(11) anode / hole injecting·transporting layer / phosphorescent-emitting layer / charge generating layer / fluorescent-emitting layer / electron injecting·transporting layer / cathode
(12) anode / hole injecting·transporting layer / fluorescent-emitting layer /electron injecting·transporting layer / charge generating layer / phosphorescent-emitting layer / cathode

In the above exemplary arrangements of the organic EL device, the monoamine derivative according to the invention and the phosphorescent material described in the first exemplary embodiment are usable for the phosphorescent-emitting layer. With this arrangement, the luminous efficiency of the organic EL device can be further enhanced and the lifetime of the organic EL device can be further prolonged. For forming the anode, hole injecting/transporting layer, electron injecting/transporting layer and cathode, the materials described in the first exemplary embodiment are usable. The fluorescent-emitting layer may be formed of a known material. The charge generating layer may be formed of a known material.

### Third Exemplary Embodiment

An organic EL device according to this exemplary embodiment includes a plurality of emitting layers and a charge blocking layer provided between any two of the plurality of emitting layers.
A preferable structure of the organic EL device according to this exemplary embodiment is exemplified by structures disclosed in Japanese Patent No. 4134280, US2007/0273270A1 and WO2008/023623A1.
Specifically, for instance, an anode, a first emitting layer, a charge blocking layer, a second emitting layer and a cathode are layered in this sequence, and an electron transporting zone having a charge blocking layer for preventing diffusion of triplet excitons is provided between the second emitting layer and the cathode. Here, the charge blocking layer is a layer for controlling the carrier injection into an emitting layer and the carrier balance between electrons and holes injected into the emitting layer by providing an energy barrier of a HOMO level or a LUMO level between adjacent emitting layers.

Examples of such an arrangement are given below.
(21) anode / hole injecting·transporting layer / first emitting layer / charge blocking layer / second emitting layer / electron injecting·transporting layer / cathode
(22) anode / hole injecting·transporting layer / first emitting layer / charge blocking layer / second emitting layer / third emitting layer / electron injecting·transporting layer / cathode

In at least one of the first emitting layer, the second emitting layer and third emitting layer, the monoamine derivative according to the invention and the phosphorescent material described in the first exemplary embodiment are usable. With this arrangement, the luminous efficiency of the organic EL device can be enhanced and the lifetime of the organic EL device can be prolonged.
In addition, when the first emitting layer emits red light, the second emitting layer emits green light, and the third emitting layer emits blue light, the entire device can emit white light.
Such an organic EL device is suitably usable as a surface light source for an illuminator, a backlight or the like.
For forming the anode, hole injecting/transporting layer, electron injecting/transporting layer and cathode, the materials described in the first exemplary embodiment are usable.
The charge blocking layer may be formed of a known material.

### Fourth Exemplary Embodiment

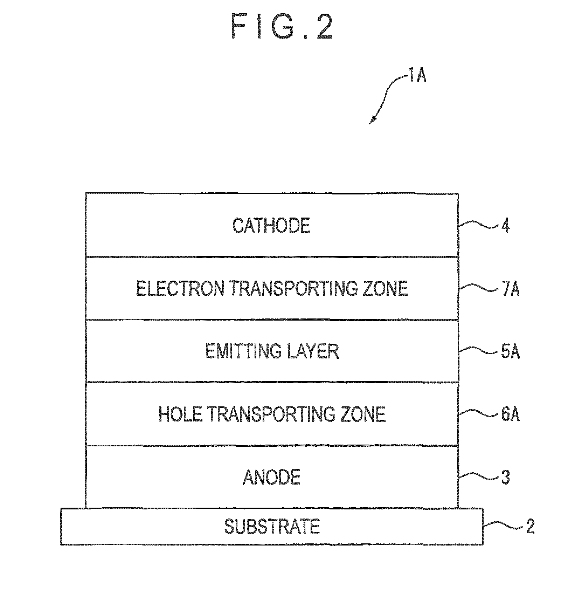

Fig. 2 schematically shows an arrangement of an organic EL device 1A according to a fourth exemplary embodiment of the invention. In the following description, the same components as those already described are denoted by the same reference signs to simplify or omit an explanation of the components.
The organic EL device 1A includes the anode 3 and the cathode 4 that are formed on the transparent substrate 2 and face each other, and a hole transporting zone 6A, an emitting layer 5A and an electron transporting zone 7A that are provided between the anode 3 and the cathode 4 and arranged in this sequence from the anode 3.

### Substrate

The transparent substrate supports the organic EL device. The substrate according to the fourth exemplary embodiment may be the same as those according to the above exemplary embodiments.

### Anode and Cathode

The anode and the cathode according to the fourth exemplary embodiment may be the same as those according to the above exemplary embodiments.

### Emitting Layer

The emitting layer according to the fourth exemplary embodiment contains a first host material, a second host material and a phosphorescent dopant (a phosphorescent material) and has the following functions (1) to (3). Here, a compound for the first host material and a compound for the second host material are different from each other.
(1) injecting function: a function for accepting, when an electrical field is applied, the holes injected by the anode or the hole transporting zone, or the electrons injected by the cathode or the electron transporting zone
(2) transporting function: a function for transporting injected electric charges (the electrons and the holes) by the force of the electrical field
(3) emitting function: a function for providing a condition for a recombination of the electrons and the holes to emit light.
Injectability of the holes may differ from that of the electrons and transporting capabilities of the hole and the electrons (represented by mobilities of the holes and the electrons) may differ from each other.

### First Host Material

The first host material may be provided by any one of known host materials, among which one having an electron transporting capability is preferable for improving the luminous efficiency and the external quantum efficiency.
The first host material having an electron transporting capability is exemplified by a substance having an electron transporting skeleton. Examples of the electron transporting skeleton are a non-fused aromatic hydrocarbon skeleton, non-fused aromatic hydrocarbon skeleton substituted with deuterium or fluorine, fused aromatic hydrocarbon skeleton, and nitrogen-containing aromatic hydrocarbon skeleton.

Examples of the non-fused aromatic hydrocarbon skeleton and the non-fused aromatic hydrocarbon skeleton substituted with a deuterium atom or a fluorine atom are the following monovalent or divalent skeletons.

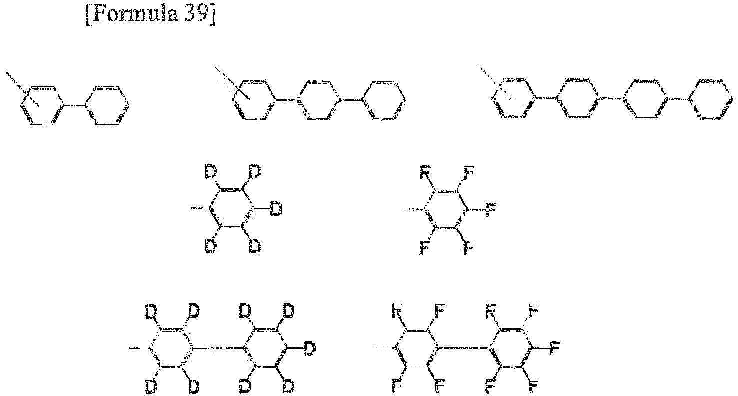

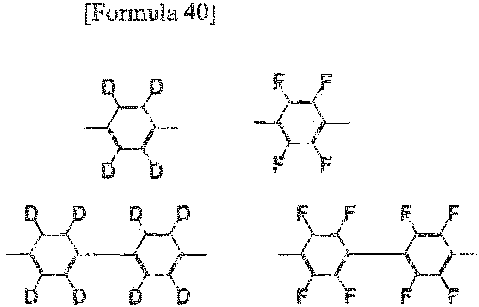

Examples of the fused aromatic hydrocarbon skeleton are as follows. The fused aromatic hydrocarbon skeleton may be monovalent or divalent. The fused aromatic hydrocarbon skeleton may be substituted with a deuterium atom or a fluorine atom. Examples of a substituent "R" used in the following fluorene skeleton are an alkyl group and aryl group.

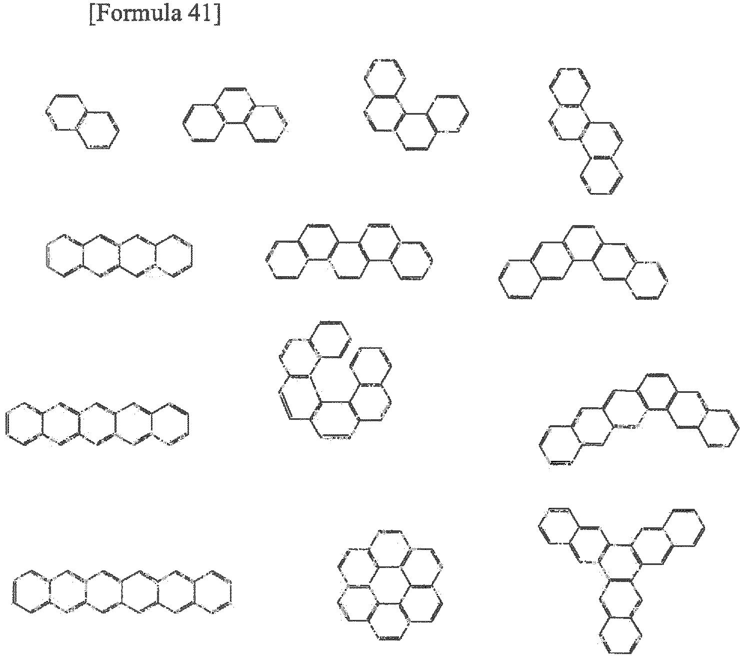

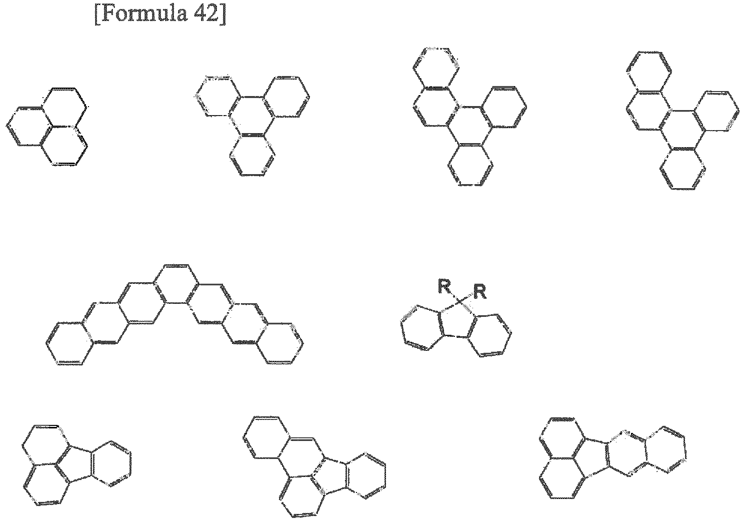

Examples of the nitrogen-containing aromatic hydrocarbon skeleton are as follows. These exemplary skeletons may be monovalent or divalent. The nitrogen-containing aromatic hydrocarbon skeleton may be substituted with a deuterium atom or a fluorine atom.

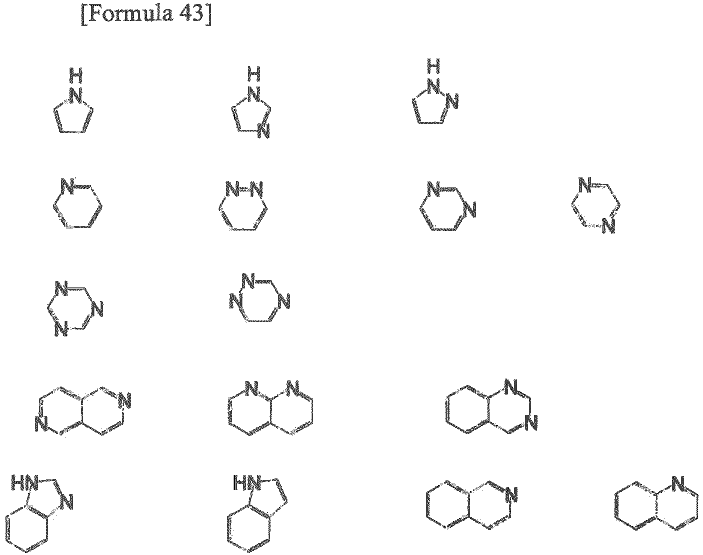

The first host material is preferably a fused aromatic compound represented by any one of the above formula (11) or formula (12).
In the formulae (11) and (12), Ar^{a}, Ar^{b}, Ar^{c}, Ar^{d}, Ar^{e}, Ar^{f} and Ar^{g} each independently represents a group derived from any one of a substituted or unsubstituted benzene skeleton, a substituted or unsubstituted naphthalene skeleton and a substituted or unsubstituted fused aromatic hydrocarbon skeleton having 3 or more rings.
When each of Ar^{a}, Ar^{b}, Ar^{c}, Ar^{d}, Ar^{e}, Ar^{f} and Ar^{g} has a substituent, the substituent is preferably an alkyl group having 1 to 20 carbon atoms, haloalkyl group having 1 to 20 carbon atoms, cycloalkyl group having 3 to 18 carbon atoms, aryl group having 6 to 30 ring carbon atoms, silyl group having 3 to 20 carbon atoms, cyano group, or halogen atom.
Examples of the alkyl group are a methyl group, ethyl group, propyl group, isopropyl group, n-butyl group, 1-methylpropyl group and 1-propylbutyl group.
The haloalkyl group is exemplified by a 2,2,2-trifluoroethyl group.
Examples of the cycloalkyl group are a cyclopropyl group, cyclobutyl group, cyclopentyl group, cyclohexyl group and cyclooctyl group.
Examples of the aryl group are the same as those for Ar¹ in the monoamine derivative.
Examples of the silyl group are a trimethylsilyl group and triethylsilyl group.
Examples of the halogen atom are fluorine, chlorine, bromine, and iodine.
When each of Ar^{a}, Ar^{b}, Ar^{c}, Ar^{d}, Ar^{e}, Ar^{f} and Ar^{g} does not have a substituent, it means that each of Ar^{a}, Ar^{b}, Ar^{c}, Ar^{d}, Ar^{e}, Ar^{f} and Ar^{g} is substituted with a hydrogen atom, which includes light hydrogen and deuterium.

Here, in the formula (11) or the formula (12), the fused aromatic hydrocarbon skeleton having 3 or more rings is preferably any one of skeletons represented by the above formulae (13) to (16).

In the formulae (13) to (16), each of Ar⁵ to Ar⁹ has a substituted or unsubstituted cyclic structure having 4 to 20 ring carbon atoms and is combined with a ring adjacent thereto to form a fused ring.

In the formula (11) or the formula (12), the fused aromatic hydrocarbon skeleton having 3 or more rings is preferably a phenanthrene skeleton, benzophenanthrene skeleton, dibenzophenanthrene skeleton, chrysene skeleton, benzochrysene skeleton, dibenzochrysene skeleton, fluoranthene skeleton, benzofluoranthene skeleton, triphenylene skeleton, benzotriphenylene skeleton, dibenzotriphenylene skeleton, picene skeleton, benzopicene skeleton, or dibenzopicene skeleton.
Among the above first host materials, the following fused aromatic hydrocarbon compounds having a fluoranthene skeleton or a phenanthrene skeleton are preferable.

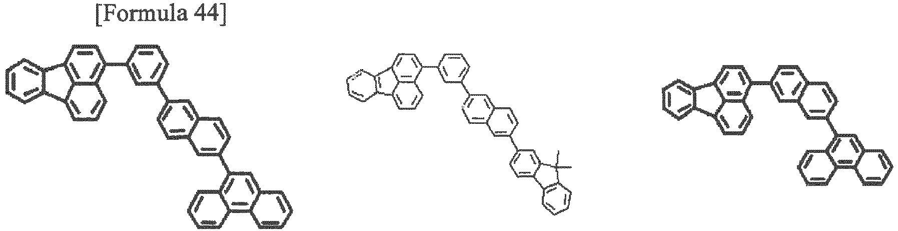

### Second Host Material

The second host material includes the monoamine derivative represented by the above formula (1).
Here, in the monoamine derivative represented by the formula (1) according to the invention, it is preferable that Ar¹ and Ar² each are a phenyl group or naphthyl group, Ar³ is a phenylene group or naphthylene group, and Ar⁴ is a benzochrysenyl group, triphenylenyl group, or phenanthrenyl group.
Examples of the second host material represented by the formula (1) according to the invention, are the same as those of the monoamine derivative. However, the invention is not limited to these exemplary compounds.

According to the invention, the monoamine derivative of the second host material is particularly preferably the following compound.

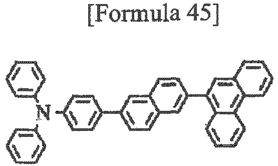

The respective triplet energies of the first host material and the second host material are preferably 2.0 eV or more.
When the compound having a triplet energy of 2.0 eV or more is used for the first host material and the second host material, it is possible to form a phosphorescent-emitting layer excellent in luminous efficiency and external quantum efficiency and suitable for mass production.
The triplet energy herein refers to a difference between an energy in the lowest triplet state and an energy in the ground state.

Even though a linear structure (e.g., an anthracene skeleton and a pentacene skeleton) or a non-linear structure having carbon atoms equal to or less than the numeral obtained by "the number of rings × 4 + 2" (e.g., a perylene skeleton and pyrene skeleton) is introduced in the molecule so as to provide the first host material and the second host material having a triplet energy of 2.0 eV or more, a sufficient triplet energy as a phosphorescent host material is unlikely to be obtained. Thus, it is preferable that such a structure is not introduced in the molecule.

For the first host material and the second host material, preferable examples of the benzophenanthrene skeleton, benzochrysene skeleton, benzofluoranthene skeleton, benzotriphenylene skeleton and benzopicene skeleton are shown below.

### Benzophenanthrene Skeleton

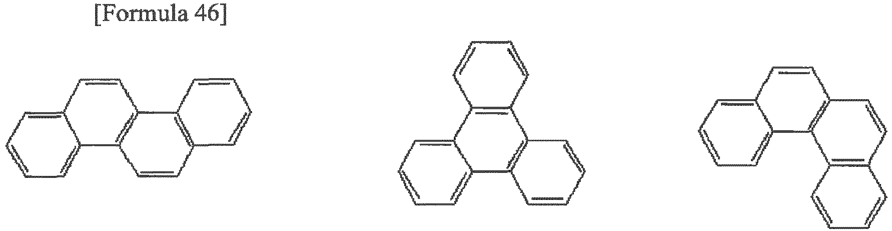

### Benzochrysene Skeleton

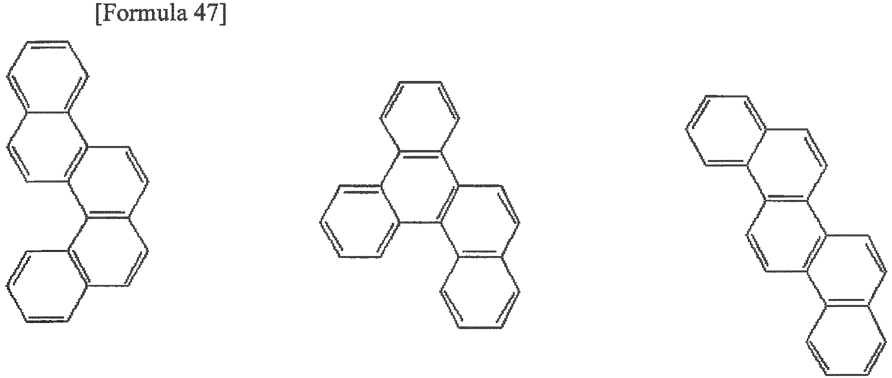

### Benzofluoranthene Skeleton

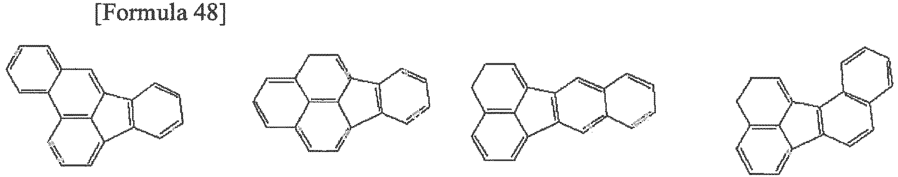

### Benzotriphenylene Skeleton

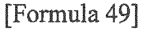

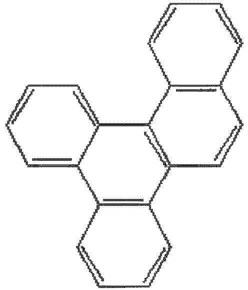

### Benzopicene Skeleton

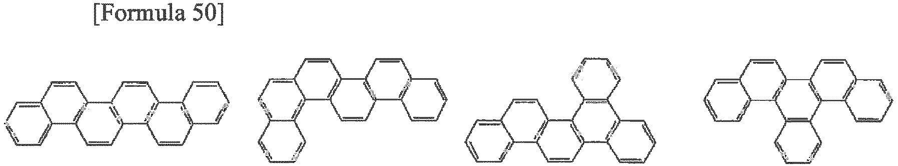

The content of the second host material in the emitting layer is preferably in a range from 5 mass% to 60 mass%, particularly preferably from 10 mass% to 50 mass%, the most preferably from 30 mass% to 50 mass%.
The thickness of the emitting layer is preferably in a range from 5 nm to 50nm, more preferably from 7 nm to 50 nm, the most preferably from 10 nm to 50 nm. When the thickness is 5 nm or more, the emitting layer can be easily formed. When the thickness is 50 nm or less, chromaticity can be easily controlled to suppress an increase in driving voltage.

According to the invention, the monoamine derivative represented by the formula (1) and containing the fused aromatic hydrocarbon skeleton having 3 or more rings is used as the second host material, so that it is possible to provide an organic EL device that exhibits excellent luminous efficiency and external quantum efficiency independently of the concentration of the second host material. Thus, it is not necessary to finely adjust the concentration of the second host material to a specific level for manufacturing an organic EL device, so that the organic EL device using the second host material according to the invention is suitable for mass production.
Even if the concentration of the phosphorescent dopant material is low, as long as the second host material according to the invention is used, it is possible to provide an organic EL device that exhibits high luminous efficiency and high external quantum efficiency independently of the concentration of the second host material.
Thus, the organic EL device according to the invention is suitable for mass production of a large-sized panel usable for an organic EL television or the like.

The first host material and the second host material, which are used for forming the emitting layer containing the phosphorescent dopant material, each have a lager triplet energy than the phosphorescent dopant material.

A method of manufacturing the second host material according to the invention is not particularly limited. The second host material can be manufactured in a known method. For instance, a coupling reaction between an amine derivative and an aromatic halogenated compound may be usable. Examples of the coupling reaction are a method using a copper catalyst described in Tetrahedron 40 (1984), pp. 435 to 1456 and a method using a palladium catalyst described in the Journal of the American Chemical Society 123 (2001), pp. 7727 to 7729.

### Phosphorescent Dopant Material

For the phosphorescent dopant material according to the fourth exemplary embodiment, the same phosphorescent material as those according to the above exemplary embodiments may be usable.

When the organic EL device includes two or more emitting layers, each of the two or more emitting layers may contain the first host material, the second host material and the phosphorescent dopant material.
A material used as the second host material according to the invention may be contained in the hole transporting zone, the electron transporting zone or the like.

### Hole Transporting Zone

The hole transporting zone, which helps injection of holes into the emitting layer, has a high hole mobility. The hole transporting zone may be provided by at least one of a hole injecting layer and a hole transporting layer, or may be provided by a single layer having hole injecting capability and hole transporting capability. The hole transporting zone may contain a blocking layer adjacent to the emitting layer.
A material for forming the hole transporting zone is preferably, for instance, an aromatic amine derivative represented by the above formula (I).
Such a compound represented by the formula (I), an example of which is given in US2009/0009067A1, paragraph [0287], is preferably the following compound (IA).

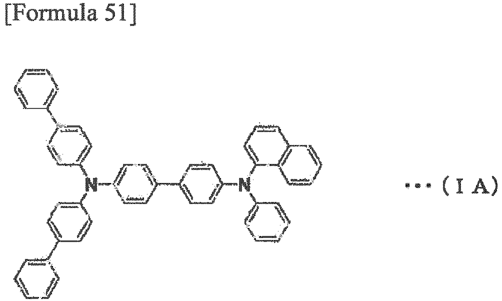

As the material for the hole transporting zone, an aromatic amine represented by the above formula (II) is also preferable.

Additionally, as the material for the hole transporting zone, a compound represented by the following formula (III) is also preferably usable.

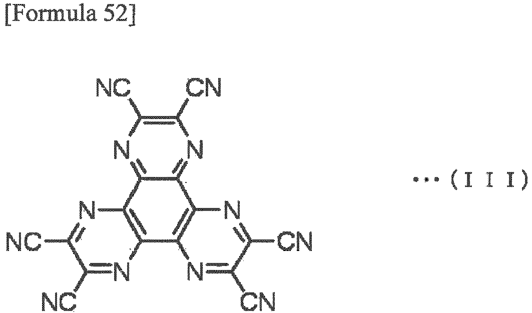

According to the invention, it is preferable that the hole injecting layer in the hole transporting zone is formed of the compound represented by the formula (III) and the hole transporting layer in the hole transporting zone is formed of the compound represented by the formula (IA).

### Electron Transporting Zone

The electron transporting zone, which helps injection of electrons into the emitting layer, has a high electron mobility. The electron transporting zone may be provided by at least one of an electron injecting layer and an electron transporting layer, or may be provided by a single layer having electron injecting capability and electron transporting capability.
A material having an electron transporting capability used for forming the electron transporting zone is preferably the same as the material for forming the electron injecting/transporting layer described in the above exemplary embodiments.

The electron transporting zone may contain the monoamine derivative according to the invention as the main component in the same manner as the electron injecting/transporting layer.
It should be noted that "as the main component" means that the monoamine derivative is contained in the electron transporting zone at a content of 50 mass% or more.
In the fourth exemplary embodiment, although the thickness of each layer other than the emitting layer is not particularly limited, the thickness is generally preferably in a range from several nanometers to 1 µm, since an extremely small thickness is likely to cause defects such as a pin hole while an extremely large thickness requires a high voltage to be applied and lowers efficiency.

In addition to the nitrogen-containing ring derivative, the material for the electron transporting zone may also be an insulator or a semiconductor (an inorganic compound) in the same manner as the electron injecting/transporting layer. When the electron transporting zone is formed of the insulator or the semiconductor, pixel defects such as a dark spot can be reduced and leakage of current can be effectively prevented, thereby improving electron injecting capability and electron transporting capability.

### Blocking Layer

The electron transporting zone may further contain a blocking layer adjacent to the emitting layer. Here, the blocking layer is adapted to control the carrier injection into an emitting layer and the carrier balance between electrons and holes injected into the emitting layer by providing an energy barrier of a HOMO level or a LUMO level between adjacent emitting layers.
With the blocking layer, holes can be trapped in the emitting layer to enhance the probability of exciton generation in the emitting layer, thereby improving the luminous efficiency and the external quantum efficiency.
As a material for the blocking layer, any known material is usable but a mixture of 8-quinolinol lithium and the following compound is preferable. The blocking layer may be formed of either one of the 8-quinolinol lithium and the following compound.

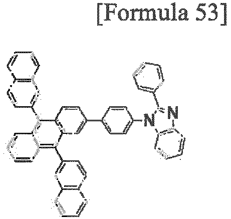

Exemplary arrangements of the organic EL device according to the invention are given below.
(1A) anode / emitting layer / electron transporting layer / electron injecting layer / cathode
(2A) anode / hole injecting layer / emitting layer / electron transporting layer /electron injecting layer / cathode
(3A) anode / hole injecting layer / hole transporting layer / emitting layer /electron transporting layer / electron injecting layer / cathode
(4A) anode / hole injecting layer / hole transporting layer / emitting layer /electron transporting layer / cathode
(5A) anode / hole injecting layer / hole transporting layer / emitting layer /blocking layer / electron injecting layer / cathode
Among the above, the exemplary arrangement (5A) is preferable.

A method of forming each layer of the organic EL device according to the invention is not particularly limited and any known method is employable.

### Fifth Exemplary Embodiment

An organic EL device according to this exemplary embodiment is a tandem device having at least two units each including an emitting layer.
In the organic EL device, for instance, a charge generating layer (also referred to as CGL) is interposed between the two units. Each unit may be provided with a hole transporting zone (a hole transporting layer and a hole injecting layer) and an electron transporting zone (an electron transporting layer and an electron injecting layer).
Exemplary tandem device structures are given below.
(11A) anode / hole injecting layer / hole transporting layer / phosphorescent-emitting layer / charge generating layer / fluorescent-emitting layer / electron transporting layer /electron injecting layer / cathode
(12A) anode / hole injecting layer / hole transporting layer / fluorescent-emitting layer /electron transporting layer / electron injecting layer / charge generating layer /phosphorescent-emitting layer / cathode

In the phosphorescent-emitting layers of the above exemplary arrangements of the organic EL device, the first host material, the monoamine derivative as the second host material and the phosphorescent dopant material according to the invention are usable. As a result, the luminous efficiency of the organic EL device can be further enhanced and the lifetime of the organic EL device can be further prolonged. For forming the anode, the hole injecting layer, the hole transporting layer, the electron injecting layer, the electron transporting layer and the cathode, the materials described in the above exemplary embodiments may be usable. In addition, for forming each of the fluorescent-emitting layer and the charge generating layer, a known material may be usable.

### Sixth Exemplary Embodiment

An organic EL device according to this exemplary embodiment includes a plurality of emitting layers and a charge blocking layer provided between any two of the plurality of emitting layers.
Preferable examples of the organic EL device according to this exemplary embodiment are given in Japanese Patent No. 4134280, US2007/0273270A1 and WO2008/023623A1.
Specifically, for instance, an anode, a first emitting layer, a charge blocking layer, a second emitting layer and a cathode are layered in this sequence, and an electron transporting zone for preventing diffusion of triplet excitons is provided between the second emitting layer and the cathode. A charge blocking layer may be contained in the electron transporting zone.

Examples of such an arrangement are given below.
(21A) anode / hole injecting layer / hole transporting layer / first emitting layer /blocking layer / second emitting layer / electron transporting layer / electron injecting layer / cathode
(22A) anode / hole injecting layer / hole transporting layer / first emitting layer /blocking layer / second emitting layer / third emitting layer / electron transporting layer /electron injecting layer / cathode

In at least one of the first emitting layer, the second emitting layer and third emitting layer, the first host material, the monoamine derivative as the second host material and the phosphorescent material according to the invention are usable. As a result, the luminous efficiency and the external quantum efficiency of the organic EL device can be enhanced.
In addition, when the first emitting layer emits red light, the second emitting layer emits green light, and the third emitting layer emits blue light, the entire device can emit white light.
Such an organic EL device is suitably usable as a surface light source for an illuminator, a backlight or the like. For forming the anode, the hole injecting layer, the hole transporting layer, the electron injecting layer, the electron transporting layer, the blocking layer and the cathode, the materials exemplified in the first exemplary embodiment may be usable.

### Modifications of Exemplary Embodiments

It should be noted that the invention is not limited to the above description but may include any modification as long as such modification is compatible with the invention.
For instance, in the invention, the emitting layer may also preferably contain an assistance substance for assisting injection of charges.
When the emitting layer is formed of a phosphorescent host material that exhibits a wide energy gap, a difference in ionization potential (Ip) between the phosphorescent host material and the hole injecting/transporting layer etc. becomes so large that it becomes difficult to inject the holes into the emitting layer and thus a driving voltage required for providing sufficient luminance may be raised.
When the emitting layer is formed of the first host material and the second host material that exhibit a wide energy gap, a difference in ionization potential (Ip) between the first and second host materials and the hole transporting zone etc. becomes so large that it becomes difficult to inject the holes into the emitting layer and thus a driving voltage required for providing sufficient luminance may be raised.

In the above instance, introducing a hole-injectable and hole-transportable assistance substance for assisting injection of charges in the emitting layer can contribute to facilitation of the injection of the holes into the emitting layer and to reduction of the driving voltage.
As the assistance substance for assisting the injection of charges, for instance, a typical hole-injectable and hole-transportable material is usable.
Examples of the material are a triazole derivative (see, for instance, the specification of US Patent No. 3,112,197), an oxadiazole derivative (see, for instance, the specification of US Patent No. 3,189,447), an imidazole derivative (see, for instance, JP-B-37-16096), a polyarylalkane derivative (see, for instance, the specifications of US Patent No. 3,615,402, No.3,820,989 and No. 3,542,544, JP-B-45-555, JP-B-51-10983, JP-A-51-93224, JP-A-55-17105, JP-A-56-4148, JP-A-55-108667, JP-A-55-156953, and JP-A-56-36656), a pyrazoline derivative and a pyrazolone derivative (see, for instance, the specifications of US Patent No. 3,180,729 and No. 4,278,746, JP-A-55-88064, JP-A-55-88065, JP-49-105537, JP-A-55-51086, JP-A-56-80051, JP-A-56-88141, JP-A-57-45545, JP-A-54-112637 and JP-A-55-74546), a phenylenediamine derivative (see, for instance, the specification of US Patent No. 3,615,404, JP-B-51-10105, JP-B-46-3712, JP-B-47-25336, JP-A-54-53435, JP-A-54-110536 and JP-A-54-119925), an arylamine derivative (see, for instance, the specifications of US Patent No. 3,567,450, No. 3,180,703, No. 3,240,597, No. 3,658,520, No. 4,232,103, No. 4,175,961 and No. 4,012,376, JP-B-49-35702, JP-B-39-27577, JP-A-55-144250, JP-A-56-119132 and JP-A-56-22437 and the specification of West Germany Patent No. 1,110,518), an amino-substituted chalcone derivative (see, for instance, the specification of US Patent No. 3,526,501), an oxazole derivative (disclosed in, for instance, the specification of US Patent No. 3,257,203), a styrylanthracene derivative (see, for instance, JP-A-56-46234), a fluorenone derivative (see, for instance, JP-A-54-110837), a hydrazone derivative (see, for instance, the specification of US Patent No. 3,717,462 and JP-A-54-59143, JP-A-55-52063, JP-A-55-52064, JP-A-55-46760, JP-A-55-85495, JP-A-57-11350, JP-A-57-148749 and JP-A-02-311591), a stilbene derivative (see, for instance, JP-A-61-210363, JP-A-61-228451, JP-A-61-14642, JP-A-61-72255, JP-A-62-47646, JP-A-62-36674, JP-A-62-10652, JP-A-62-30255, JP-A-60-93455, JP-A-60-94462, JP-A-60-174749 and JP-A-60-175052), a silazane derivative (see the specification of US Patent No. 4,950,950), a polysilane type (see JP-A-02-204996), an aniline-based copolymer (see JP-A-02-282263), and a conductive polymer oligomer (particularly, thiophene oligomer) disclosed in JP-A-01-211399.

The hole-injectable and hole-transportable material, examples of which are as listed above, is preferably a porphyrin compound (disclosed in JP-A-63-295695 etc.), an aromatic tertiary amine compound or a styrylamine compound (see, for instance, the specification of US Patent No. 4,127,412, JP-A-53-27033, JP-A-54-58445, JP-A-54-149634, JP-A-54-64299, JP-A-55-79450, JP-A-55-144250, JP-A-56-119132, JP-A-61-295558, JP-A-61-98353 or JP-A-63-295695), particularly preferably an aromatic tertiary amine compound.

In addition, a compound having two fused aromatic rings in the molecule as disclosed in US Patent No. 5,061,569 is also preferable. Examples of such a compound are 4,4'-bis(N-(1-naphthyl)-N-phenylamino)biphenyl (hereinafter, abbreviated as NPD), and 4,4',4"-tris(N-(3-methylphenyl)-N-phenylamino)triphenylamine in which three triphenylamine units are bonded in a starbust form as disclosed in JP-A-04-308688.
Further, a hexaazatriphenylene derivative disclosed in Japanese Patent No. 3614405 and No. 3571977 and US Patent No. 4,780,536 may also preferably be used as the hole-injectable and hole-transportable material.
Alternatively, inorganic compounds such as p-type Si and p-type SiC may also be used as the hole injecting material.

The organic EL device according to the invention may include a reduction-causing dopant provided in an interfacial region between the cathode and the electron injecting/transporting layer. The organic EL device can thus emit light with enhanced luminance intensity and have a longer lifetime. The reduction-causing dopant may be at least one compound selected from an alkali metal, an alkali metal complex, an alkali metal compound, an alkaline earth metal, an alkaline earth metal complex, an alkaline earth metal compound, a rare-earth metal, a rare-earth metal complex, a rare-earth metal compound and the like.

The amine derivative according to the invention is exemplarily applied to the organic EL device, but may be applied to an organic electronic device for an organic solar cell, an organic semiconductor laser, a sensor using an organic substance and an organic TFT.

It should be noted that the "hole injecting/transporting layer (or hole injecting·transporting layer)" herein means "at least either one of a hole injecting layer and a hole transporting layer" while the "electron injecting/transporting layer (or electron injecting·transporting layer)" herein means "at least either one of an electron injecting layer and an electron transporting layer".

### Examples

Next, the invention will be described in further detail with reference to Example(s) and Comparative(s). However, the invention is not limited by the description of Example(s).
In Example(s) and Comparative(s), the following materials were used as the phosphorescent host material, the phosphorescent material, the material for forming the hole injecting/transporting layer, and the material for forming the electron transporting layer.

### Phosphorescent Host Material

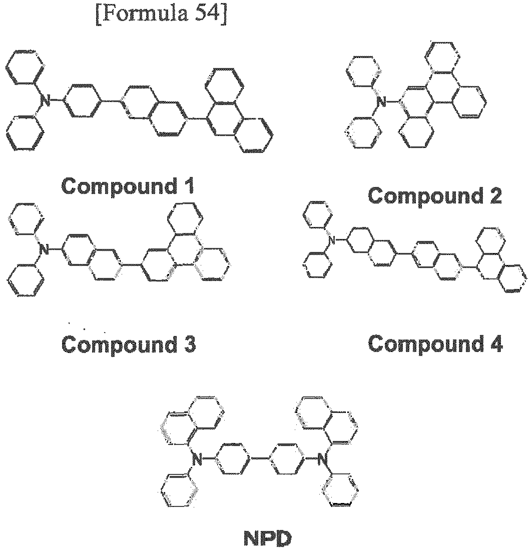

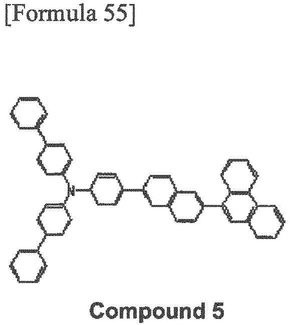

### Phosphorescent Material

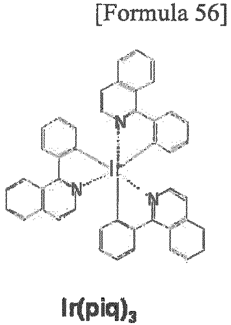

### Material for Hole Injecting/Transporting Layer and Material for Electron Transporting Layer

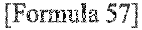

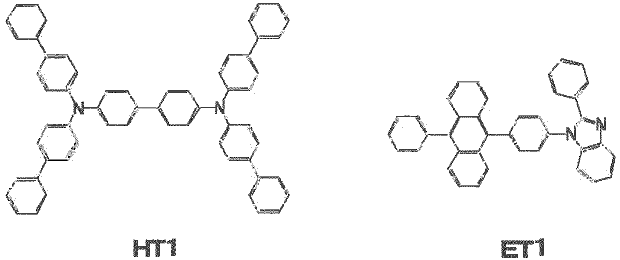

### Synthesis Example 1

### Synthesis of Compound 1

Compound 1 was synthesized in the following process. Compounds 2 to 4 were synthesized in the same manner as Compound 1.

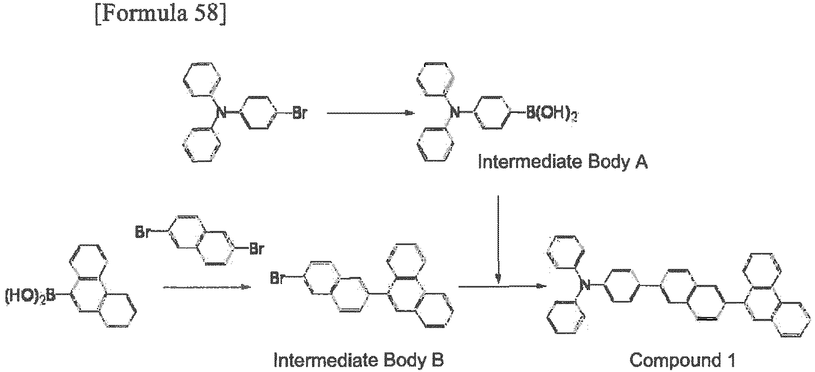

### Synthesis Example (1-1) Synthesis of Intermediate Body A

20.7 g (64.0 mmol) of (4-bromo-phenyl)-diphenylamine was dissolved in 400 mL of dehydrated tetrahydrofuran and 300 mL of dehydrated toluene, cooled down to -70 degrees C, dropped with 44.6 mL (70.4 mmol) of n-butyllithium, and stirred for one hour. The mixture was added with 44.0 mL (192 mmol) of triisopropyl boronate ester and then the temperature of the mixture was raised to room temperature in two hours. The mixture was added with 200 mL of 10% hydrochloric acid and stirred for two hours. The resulting precipitate was separated by filtration, cleaned with toluene, and dried under reduced pressure, by which a faint yellow intermediate body A (16.3 g, yield 88%) was obtained.

### Synthesis Example (1-2) Synthesis of Intermediate Body B

Under an argon atmosphere, 11.1 g (50.0 mmol) of 9-phenanthrene boronic acid, 14.3 g (50.0 mmol) of 2,6-dibromonaphthalene, 1.73 g (1.5 mmol) of tetrakis(triphenylphosphine)palladium(0), 150 mL of 1,2-dimethoxyethane, and 75 mL of 2M sodium hydrogencarbonate solution were mixed, and stirred for eight hours while being refluxed. The reaction mixture was then cooled down to room temperature, added with water, and stirred for one hour. The resulting solid was separated by filtration, cleaned with water and methanol, and dried under reduced pressure. The solid was then refined by silica-gel chromatography, by which a faint yellow solid (15.9 g, yield 83%) was obtained.

### Synthesis Example (1-3) Synthesis of Compound 1

Under an argon atmosphere, 3.83 g (10.0 mmol) of the intermediate body B, 3.04 g (10.5 mmol) of the intermediate body A, 0.35 g (0.30 mmol) of tetrakis(triphenylphosphine)palladium(0), 30 mL of 1,2-dimethoxyethane, and 15 mL of 2M sodium hydrogencarbonate solution were mixed, and stirred for eight hours while being refluxed.
The reaction mixture was then cooled down to room temperature, added with water, and stirred for one hour. The resulting solid was separated by filtration, cleaned with water and methanol, and dried under reduced pressure. The solid was then refined by silica-gel chromatography, by which a yellow solid (4.30 g, yield 78.5%) was obtained.

The obtained compound was analyzed by HPLC (High Performance Liquid Chromatography) and FD-MS (Field Desorption ionization-Mass Spectrometry). The results of the analysis are shown below.
HPLC: purity 99.9%
FD-MS: calcd for C₄₂H₂₉N = 547.69, found m/z = 548(M⁺,100))

### Synthesis Example 2

### Synthesis of Compound 5

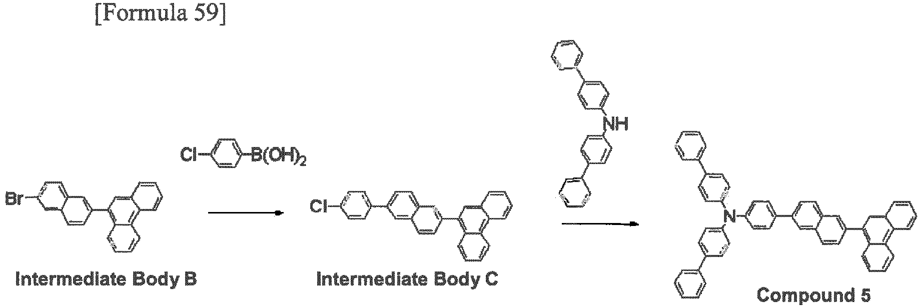

### Synthesis Example (2-1) Synthesis of Intermediate Body C

Under an argon atmosphere, 3.8 g (10 mmol) of the intermediate body B, 1.56 g (10 mmol) of 4-chlorophenyl boronic acid, 0.23 g (0.2 mmol) of tetrakis(triphenylphosphine)palladium, 30 mL of toluene, and 15 mL of 2M sodium carbonate solution were mixed, and stirred for eight hours while being refluxed. The reaction mixture was then cooled down to room temperature, added with water, and stirred for one hour. The resulting solid was separated by filtration and cleaned with water and methanol. The solid was then refined by silica-gel chromatography, by which an intermediate body C (3.1 g, yield 75%) was obtained.

### Synthesis Example (2-2) Synthesis of Compound 5

Under an argon atmosphere, 3 g (7.5 mmol) of the intermediate body C,
2.56 g (8 mmol) of bis(4-biphenyl)amine, 0.14 g (0.15 mmol) of tris(dibenzylideneacetone)dipalladium(0), 0.17 g (0.6 mmol) of tri-tert-butylphosphonium tetrafluoroborate, and 1.0 g (10.5 mmol) of sodium-tert-butoxide were mixed, and stirred for eight hours while being refluxed.
The reaction mixture was added with water and stirred for one hour. The resulting solid was separated by filtration and cleaned with water and methanol. The solid was then refined by silica-gel chromatography, by which a target substance (3.65 g, yield 70%) was obtained.
The obtained compound was analyzed by HPLC (High Performance Liquid Chromatography) and FD-MS (Field Desorption ionization-Mass Spectrometry). The results of the analysis are shown below.
HPLC: purity 99.9%
FD-MS: calcd for C₅₄H₃₇N= 699.29, found m/z = 699(M⁺,100))

### Example 1: Manufacture of Organic EL Device

A glass substrate (size: 25 mm × 75 mm × 0.7 mm thick) having an ITO transparent electrode (manufactured by Asahi Glass Co., Ltd) was ultrasonic-cleaned in isopropyl alcohol for five minutes, and then UV/ozone-cleaned for 30 minutes. The ITO transparent electrode serves as the anode.
After the glass substrate having the transparent electrode line was cleaned, the glass substrate was mounted on a substrate holder of a vacuum deposition apparatus. A 50-nm thick film of Compound HT1 was initially formed on a surface of the glass substrate where the transparent electrode line was provided so as to cover the transparent electrode. The film of Compound HT1 serves as the hole injecting/transporting layer.
Subsequently, on the film of the hole injecting/transporting layer, Compound 1 and Compound Ir(piq)₃ (the phosphorescent material) were co-evaporated by resistance heating deposition to form a 40-nm thick film.
The content of Compound Ir(piq)₃ was set at 10 mass%. The formed film serves as the emitting layer (the phosphorescent-emitting layer). The emission wavelength of Compound Ir(piq)₃ is 629 nm.
After the formation of the film of the emitting layer, Compound ET1 was deposited on the emitting layer to form a 40-nm thick film. The film of Compound ET1 serves as the electron transporting layer.
Subsequently, a 1-nm thick film was formed at a film-forming speed of 0.1 nm/min. The LiF film serves as an electron-injectable electrode (cathode).
After the formation of the LiF layer, a metal A1 was deposited on the LiF layer to form an 80-nm thick metal cathode.
As a result, the organic EL device of Example 1 was manufactured.

### Examples 2 to 5 and Comparative 1

The respective organic EL devices of Examples 2 to 5 and Comparative 1 were manufactured in the same manner as that of Example 1 except that Compounds 2 to 5 and NPD were used as the phosphorescent host material. The manufactured organic EL devices were evaluated as follows.

### Evaluation of Organic EL Device

Each of the manufactured organic EL devices of Examples and Comparative was driven by direct current to emit light so as to measure luminous efficiency at a current density of 10 mA/cm², external quantum efficiency, and time elapsed until the initial luminance intensity of 2600 cd/m² decreased to 80%. The results of the measurement are shown in Table 1.

**Table 1**

| | Phosphorescent Host Material | Driving Voltage (V) | Luminous Efficiency L/J (cd/A) | External Quantum Efficiency EQE (%) | Life time LT80 (h) |
|---|---|---|---|---|---|
| Example 1 | Compound 1 | 4.7 | 10.6 | 12.7 | 350 |
| Example 2 | Compound 2 | 4.9 | 9.6 | 11.5 | 330 |
| Example 3 | Compound 3 | 4.8 | 10.4 | 12.5 | 340 |
| Example 4 | Compound 4 | 4.6 | 10.9 | 13.1 | 360 |
| Example 5 | Compound 5 | 4.8 | 10.5 | 12.5 | 400 |
| Comparative 1 | NPD | 5.3 | 4.6 | 5.5 | 30 |

As is apparent from the results shown in Table 1, it has been found out that in comparison with the organic EL device of Comparative 1 using an amine derivative such as NPD, the organic EL devices of Examples, each of which uses the monoamine derivative having a specific structure, can have improved luminous efficiency, external quantum efficiency and lifetime while being driven by a low driving voltage.

Next, the invention will be described in further detail with reference to Example(s) and Comparative(s) of the organic EL device in which the first host material and the second host material are used in the emitting layer. However, the invention is not limited by the description of Example(s).
In Example(s) and Comparative(s), the following materials were used as the first host material, the second host material, the phosphorescent dopant material, and the materials of the hole injecting layer, the hole transporting layer, the blocking layer and the electron injecting layer.

### First Host Material

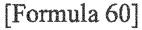

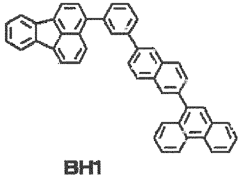

### Second Host Material

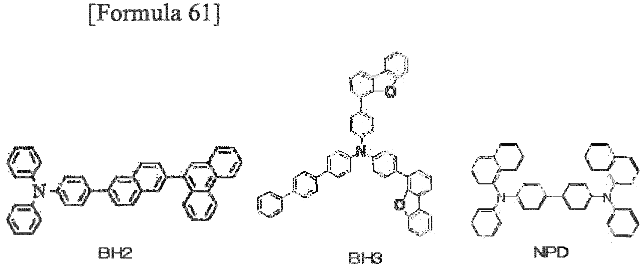

### Phosphorescent Dopant Material

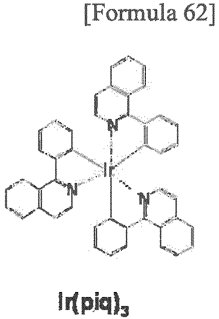

### Materials for Hole Injecting Layer, Hole Transporting Layer, Blocking Layer and Electron Injecting Layer

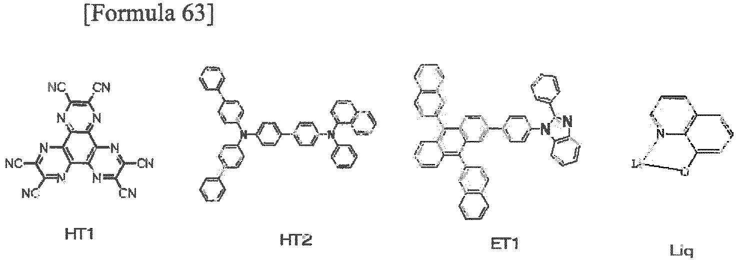

### Synthesis Example 3

### Synthesis of Compound BH2

Compound BH2 was synthesized in the following process.

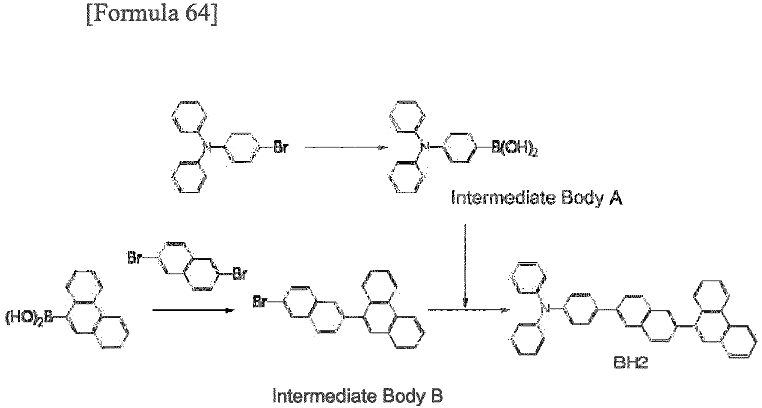

### Synthesis Example (1-1) Synthesis of Intermediate Body A

20.7 g (64.0 mmol) of (4-bromo-phenyl)-diphenylamine was dissolved in 400 mL of dehydrated tetrahydrofuran and 300 mL of dehydrated toluene, cooled down to -70 degrees C, dropped with 44.6 mL (70.4 mmol) of n-butyllithium, and stirred for one hour. The mixture was added with 44.0 mL (192 mmol) of triisopropyl boronate ester and then the temperature of the mixture was raised to room temperature in two hours. The mixture was added with 200 mL of 10% hydrochloric acid and stirred for two hours. The resulting precipitate was separated by filtration, cleaned with toluene, and dried under reduced pressure, by which a faint yellow intermediate body A (16.3 g, yield 88%) was obtained.

### Synthesis Example (1-2) Synthesis of Intermediate Body B

Under an argon atmosphere, 11.1 g (50.0 mmol) of 9-phenanthrene boronic acid, 14.3 g (50.0 mmol) of 2,6-dibromonaphthalene, 1.73 g (1.5 mmol) of tetrakis(triphenylphosphine)palladium(0), 150 mL of 1,2-dimethoxyethane, and 75 mL of 2M sodium hydrogencarbonate solution were mixed, and stirred for eight hours while being refluxed.
The reaction mixture was then cooled down to room temperature, added with water, and stirred for one hour. The resulting solid was separated by filtration, cleaned with water and methanol, and dried under reduced pressure. The solid was then refined by silica-gel chromatography, by which a faint yellow solid (the intermediate body B) (15.9 g, yield 83%) was obtained.

### Synthesis Example (1-3) Synthesis of Compound BH2

Under an argon atmosphere, 3.83 g (10.0 mmol) of the intermediate body B, 3.04 g (10.5 mmol) of the intermediate body A, 0.35 g (0.30 mmol) of tetrakis(triphenylphosphine)palladium(0), 30 mL of 1,2-dimethoxyethane, and 15 mL of 2M sodium hydrogencarbonate solution were mixed, and stirred for eight hours while being refluxed.
The reaction mixture was then cooled down to room temperature, added with water, and stirred for one hour. The resulting solid was separated by filtration, cleaned with water and methanol, and dried under reduced pressure. The solid was then refined by silica-gel chromatography, by which a yellow solid (4.30 g, yield 78.5%) was obtained.

The obtained compound was analyzed by HPLC (High Performance Liquid Chromatography) and FD-MS (Field Desorption ionization-Mass Spectrometry). The results of the analysis are shown below. From the results of the analysis, it was confirmed that the obtained compound was Compound BH2.
HPLC: purity 99.9%
FD-MS: calcd for C₄₂H₂₉N = 547.69, found m/z = 548(M⁺,100))

### Triplet Energy of Second Host Material

The triplet energies of BH2, NPD and BH3 (the second host material) are 2.48 eV, 2.46 eV and 2.58 eV, respectively.
A triplet energy (EgT) of the second host material was measured by a known phosphorescence measurement method (e.g., a method described on and near page 50 of "Hikarikagaku no Sekai" (edited by The Chemical Society of Japan, 1993)). Specifically, the second host material was dissolved in a solvent (sample: 10 µmol/L, EPA (diethylether : isopentane : ethanol = 5:5:2 in volume ratio, each solvent is in a spectroscopic grade) to provide a sample for phosphorescence measurement. The sample set in a quartz cell was cooled down to 77 K and irradiated with an excitation light. The resulting phosphorescence was measured relative to a wavelength. A tangent line was drawn to be tangent to a rising section of the phosphorescence spectrum on the short-wavelength side, and the obtained wavelength value was converted into an energy value, which was defined as EgT. For the measurement, the body of the F-4500 fluorescence spectrophotometer (manufactured by Hitachi, Ltd.) and optional accessories for low-temperature measurement were used. In place of the above measuring device, a cooling device, a low-temperature container, an excitation light source and a light-receiving device may be used in combination for the measurement. According to the invention, the following expression was used for conversion of the wavelength.
conversion expression: EgT (eV) = 1239.85 / λedge
When the phosphorescence spectrum is expressed in coordinates, the ordinate axis of which indicates the phosphorescence intensity and the abscissa axis of which indicates the wavelength, and a tangent is drawn to a rising section of the phosphorescence spectrum on the short-wavelength side, "λedge" is a wavelength value at the intersection of the tangent and the abscissa axis. unit: nm

### Example 6: Manufacture of Organic EL Device

The organic EL device according to Example 6 was manufactured as follows.
A glass substrate (size: 25 mm × 75 mm × 1.1 mm thick, manufactured by Geomatec Co., Ltd.) having an ITO transparent electrode (anode) was ultrasonic-cleaned in isopropyl alcohol for five minutes, and then UV/ozone-cleaned for 30 minutes. After the glass substrate having the transparent electrode line was cleaned, the glass substrate was mounted on a substrate holder of a vacuum deposition apparatus. Compound HTI was initially formed on a surface of the glass substrate where the transparent electrode line was provided so as to cover the transparent electrode. As a result, a 5-nm thick hole injecting layer was formed.
Compound HT2 was deposited on the hole injecting layer to form a 205-nm thick hole transporting layer. In this manner, the hole transporting zone provided by the hole injecting layer and the hole transporting layer was formed.
Compound BH1 (the first host material) and Compound BH2 (the second host material) and Ir(piq)₃ (the phosphorescent dopant material) were co-evaporated on the hole transporting zone. As a result, a 45-nm thick emitting layer enabling phosphorescent emission was formed. In the emitting layer, the concentration of Ir(piq)₃ was set at 8 mass% and the concentration of the second host material was 10 mass%.
Next, Compound ET1 and Compound Liq (a metal complex containing an alkali metal) were co-evaporated on the emitting layer. As a result, a 20-nm thick blocking layer was formed. The concentration of Compound Liq in the blocking layer was set at 50 mass%.
Compound Liq was deposited on the blocking layer to form a 1-nm thick electron injecting layer. In this manner, the electron transporting zone provided by the blocking layer and the electron injecting layer was formed.
A metal aluminum (Al) was deposited on the electron transporting zone to form an 80-nm thick cathode.
Thus, the organic EL device of Example 6 was manufactured.

### Examples 7 to 12 and Comparatives 2 to 9

The respective organic EL devices of Examples 7 to 12 and Comparatives 2 to 9 were manufactured in the same manner as that of Example 6 except that a compound used as the second host material and the respective concentrations of the second host material and the phosphorescent dopant material were changed as shown in Table 2. The second host material was not used for Comparatives 7 to 9.
The manufactured organic EL devices were evaluated as follows.

### Evaluation of Organic EL Device

The manufactured organic EL devices of Examples 7 to 12 and Comparatives 2 to 9 were driven by direct current to emit light, and then the luminous efficiency at a current density of 1 mA/cm² and the external quantum efficiency were measured. The results of the measurement are shown in Table 2.

**Table 2**

| | Second Host Material (Concentration:mass%) | Phosphorescent Dopant Material Concentration (mass%) | Driving Voltage (V) | Luminous Efficiency L/J (cd/A) | External Quantum Efficiency EQE (%) |
|---|---|---|---|---|---|
| Example 6 | BH2 (10) | 8 | 3.50 | 14.60 | 16.34 |
| Example 7 | BH2 (30) | 8 | 3.41 | 15.20 | 16.70 |
| Example 8 | BH2 (50) | 8 | 3.29 | 16.04 | 17.00 |
| Example 9 | BH2 (30) | 1 | 3.60 | 17.90 | 17.31 |
| Example 10 | BH2 (50) | 1 | 3.53 | 17.11 | 16.04 |
| Example 11 | BH2 (30) | 3 | 3.68 | 15.08 | 16.89 |
| Example 12 | BH2 (50) | 3 | 3.07 | 15.54 | 16.81 |
| Comparative 2 | NPD (10) | 8 | 3.30 | 13.97 | 15.64 |
| Comparative 3 | NPD (30) | 8 | 3.36 | 10.46 | 11.18 |
| Comparative 4 | NPD (50) | 8 | 3.35 | 7.66 | 8.16 |
| Comparative | BH3 (10) | 8 | 3.29 | 14.36 | 17.05 |
| Comparative 6 | BH3 (30) | 8 | 3.19 | 14.18 | 15.77 |
| C om Comparative 7 | - | 1 | 3.72 | 13.64 | 14.77 |
| Comparative 8 | - | 3 | 3.48 | 13.30 | 15.13 |
| Comparative 9 | - | 8 | 3.39 | 14.12 | 16.57 |

Referring to the results regarding Examples 6 to 8 shown in Table 2, it has been found out that even when the concentration of the second host material is changed, the luminous efficiency and the external quantum efficiency are almost unchanged and thus each kept at a high level. Referring to the results regarding Examples 9 to 12, it has been found out that even when the concentration of the phosphorescent dopant material is changed, the luminous efficiency and the external quantum efficiency are almost independent of the concentration of the second host material. In view of the above, it has been found out that when the monoamine derivative represented by the formula (1) and containing the fused aromatic hydrocarbon skeleton having 3 or more rings is used as the second host material, it is possible to provide an organic EL device that is excellent in luminous efficiency and external quantum efficiency and is suitable for mass production.
Referring to the results regarding to Comparatives 2 to 4 and Comparatives 5 and 6, it has been found out that when the diamine derivative or the monoamine derivative having no fused aromatic hydrocarbon skeleton is used as the second host material, in particular, the luminous efficiency is lowered and the luminous efficiency and the external quantum efficiency are significantly changed depending on the concentration of the second host material. In view of the above, it has been found out that when the emitting layer of an organic EL device contains the first host material and the second host material and the second host material is provided by the diamine derivative (NPD) or the monoamine derivative (Compound BH3) as in Comparatives 2 to 6, the organic EL device is not suitable for mass production.
Referring to the results regarding Comparatives 7 to 9, it has been found out that when the host material of the emitting layer is provided only by Compound BH1 that is structurally different from the monoamine derivative according to the invention and the second host material is not provided by the monoamine derivative according to the invention, the luminous efficiency and the external quantum efficiency are lowered and these efficiencies are significantly dependent on the concentration of the phosphorescent dopant material. In view of the above, it has been found out that an organic EL device in which the second host material is not provided by the monoamine derivative according to the invention is also not suitable for mass production.

### Examples 13 and 14: Manufacture of Organic EL Device

The respective organic EL devices of Examples 13 and 14 were manufactured in the same manner as that of Example 6 except that a compound used as the second host material and the respective concentrations of the second host material and the phosphorescent dopant material were changed as shown in Table 3.
Compound BH4 used as the second host material of each of Examples 13 and 14 is shown below.

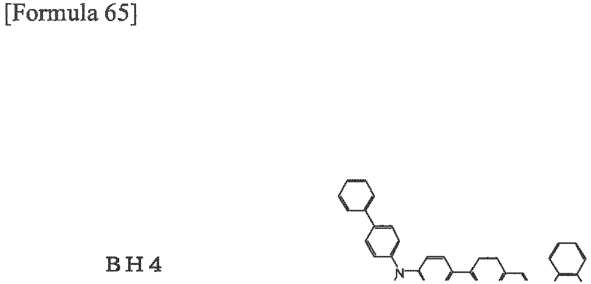

The luminous efficiency and the external quantum efficiency of each of these organic EL devices were measured in the same manner as those of the organic EL device of Example 6. The results of the measurement are shown in Table 3.

**Table 3**

| | Second Host Material (Concentration: mass%) | Phosphorescent Dopant Material Concentration (mass%) | Driving Voltage (V) | Luminous Efficiency L/J (cd/A) | External Quantum Efficiency EQE (%) |
|---|---|---|---|---|---|
| Example 13 | BH4 (30) | 8 | 3.55 | 14.40 | 16.20 |
| Example 14 | BH4 (50) | 8 | 3.47 | 14.65 | 16.35 |

### INDUSTRIAL APPLICABILITY

A monoamine derivative according to the invention is usable to provide an organic EL device excellent in luminous efficiency, external quantum efficiency and emission lifetime.
The organic EL device according to the invention, which is excellent in luminous efficiency and external quantum efficiency and suitable for mass production, is applicable to a large-sized organic EL television or the like.

### EXPLANATION OF CODES

| | |
|---|---|
| 1 | organic electroluminescence device |
| 1A | organic electroluminescence device |
| 2 | transparent substrate |
| 3 | anode |
| 4 | cathode |
| 5 | emitting layer |
| 5A | emitting layer |
| 6 | hole injecting/transporting layer |
| 6A | hole transporting zone |
| 7 | electron injecting/transporting layer |
| 7A | electron transporting zone |
| 10 | organic thin-film layer |

## Claims

1. An organic electroluminescence device comprising:
an anode;
a cathode being opposed to the anode; and
an emitting layer being provided between the anode and the cathode, wherein
the emitting layer comprises a host material and a phosphorescent dopant material, and
the host material comprises a monoamine derivative represented by a formula (1A) below, 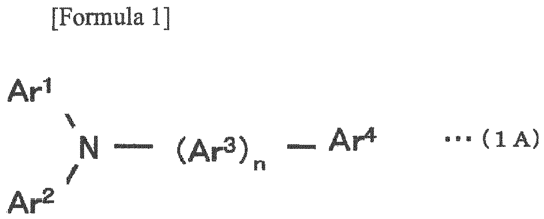 where each of Ar¹, Ar² and Ar⁴ is a substituted or unsubstituted aryl group or heteroaryl group,
Ar³ is a substituted or unsubstituted arylene group or heteroarylene group,
n is an integer of 0 to 5,
when n is 2 or more, Ar³ is allowed to be mutually the same or different, and
at least one of Ar¹, Ar², Ar³ and Ar⁴ is a group derived from a fused aromatic hydrocarbon skeleton having 3 or more rings.

2. The organic electroluminescence device according to claim 1, wherein the fused aromatic hydrocarbon skeleton having 3 or more rings is any one of skeletons represented by formulae (2A) to (5A) below, 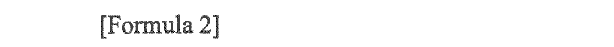 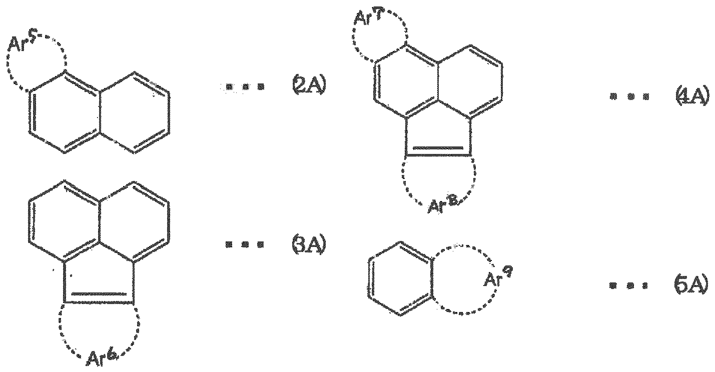 where each of Ar⁵ to Ar⁹ has a substituted or unsubstituted cyclic structure having 4 to 20 ring carbon atoms and is combined with a ring adjacent thereto to form a fused ring.

3. The organic electroluminescence device according to claim 1 or 2, wherein
the emitting layer comprises:
a first host material;
a second host material; and
a phosphorescent dopant material, and
the second host material is the monoamine derivative.

4. The organic electroluminescence device according to claim 3, wherein Ar⁴ in the formula (1A) for the second host material is a group derived from the fused aromatic hydrocarbon skeleton having 3 or more rings.

5. The organic electroluminescence device according to claim 3, wherein Ar⁴ in the formula (1A) for the second host material is a monovalent group derived from any one of skeletons below:
a phenanthrene skeleton;
a benzophenanthrene skeleton;
a dibenzophenanthrene skeleton;
a chrysene skeleton;
a benzochrysene skeleton;
a dibenzochrysene skeleton;
a fluoranthene skeleton;
a benzofluoranthene skeleton;
a triphenylene skeleton;
a benzotriphenylene skeleton;
a dibenzotriphenylene skeleton;
a picene skeleton;
a benzopicene skeleton; and
a dibenzopicene skeleton.

6. The organic electroluminescence device according to any one of claims 1 to 5, wherein the phosphorescent dopant material is an ortho-metalated complex of a metal selected from iridium (Ir), osmium (Os) and platinum (Pt).

7. A monoamine derivative represented by a formula (1) below, 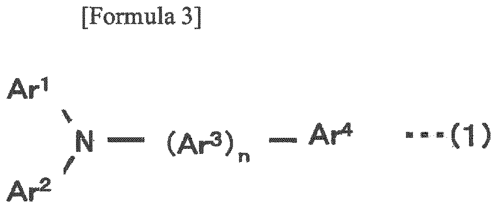 where each of Ar¹, Ar² and Ar⁴ is a substituted or unsubstituted aryl group or heteroaryl group,
Ar³ is a substituted or unsubstituted arylene group or heteroarylene group,
n is an integer of 0 to 5,
when n is 0, at least one of Ar¹, Ar² and Ar⁴ is a group derived from a fused aromatic hydrocarbon skeleton having 3 or more rings,
when n is 1, at least one of Ar¹, Ar², Ar³ and Ar⁴ is a group derived from the fused aromatic hydrocarbon skeleton having 3 or more rings, and
when n is 2 or more, Ar³ is allowed to be mutually the same or different.

8. The monoamine derivative according to claim 7, wherein when n is 2 or more in the formula (1), at least one of Ar¹, Ar² and Ar⁴ is a group derived from the fused aromatic hydrocarbon skeleton having 3 or more rings.

9. The monoamine derivative according to claim 7 or 8, wherein the fused aromatic hydrocarbon skeleton having 3 or more rings is any one of skeletons represented by formulae (2) to (5) below, 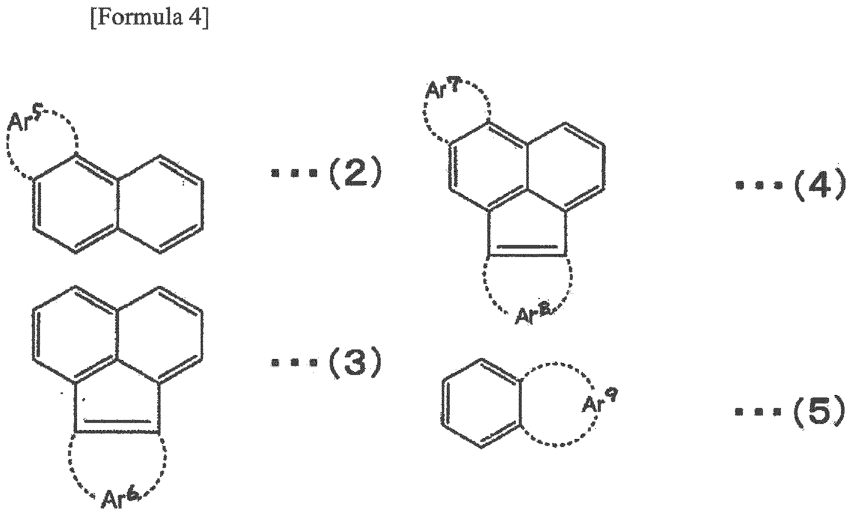 where each of Ar⁵, Ar⁶, Ar⁷, Ar⁸ and Ar⁹ has a substituted or unsubstituted cyclic structure having 4 to 20 ring carbon atoms and is combined with a ring adjacent thereto to form a fused ring.

10. The monoamine derivative according to claim 9, wherein Ar⁴ in the formula (1) is a monovalent group derived from any one of the skeletons represented by the formulae (2) to (5).

11. The monoamine derivative according to claim 10, wherein Ar⁴ in the formula (1) is a monovalent group derived from any one of skeletons below:
a phenanthrene skeleton;
a benzophenanthrene skeleton;
a dibenzophenanthrene skeleton;
a chrysene skeleton;
a benzochrysene skeleton;
a dibenzochrysene skeleton;
a fluoranthene skeleton;
a benzofluoranthene skeleton;
a triphenylene skeleton;
a benzotriphenylene skeleton;
a dibenzotriphenylene skeleton;
a picene skeleton;
a benzopicene skeleton; and
a dibenzopicene skeleton.

12. The monoamine derivative according to claim 7, wherein when n is 0, the monoamine derivative is represented by a formula (6) below, and
at least one of Ar¹, Ar² and Ar⁴ is a fused aromatic hydrocarbon group selected from a group consisting of:
a phenanthrenyl group;
a benzophenanthrenyl group;
a dibenzophenanthrenyl group;
a benzochrysenyl group;
a dibenzochrysenyl group;
a fluoranthenyl group;
a benzofluoranthenyl group;
a triphenylenyl group;
a benzotriphenylenyl group;
a dibenzotriphenylenyl group;
a picenyl group;
a benzopicenyl group;
a dibenzopicenyl group;
a phenalenyl group;
an acenaphthenyl group; and
a diazaphenanthrenyl group.

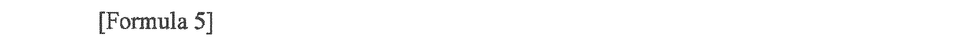

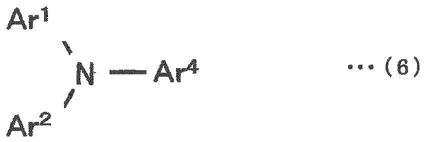

13. The monoamine derivative according to claim 7, wherein when n is 1, the monoamine derivative is represented by a formula (7) below,
Ar⁴ is a fused aromatic hydrocarbon group selected from a group consisting of:
a phenanthrenyl group;
a benzophenanthrenyl group;
a dibenzophenanthrenyl group;
a benzochrysenyl group;
a dibenzochrysenyl group;
a fluoranthenyl group;
a benzofluoranthenyl group;
a triphenylenyl group;
a benzotriphenylenyl group;
a dibenzotriphenylenyl group;
a picenyl group;
a benzopicenyl group; and
a dibenzopicenyl group, and
when Ar³ is a phenylene group or 1,4-naphthylene group, Ar⁴ is not a phenanthrenyl group bonded to Ar³ at 2,3,9-positions.

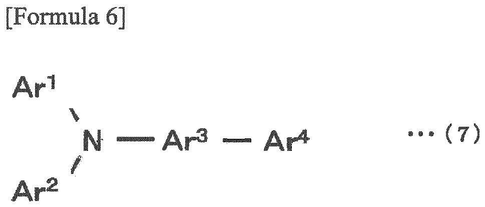

14. The monoamine derivative according to claim 7, wherein
when n is 2 or more, Ar⁴ is an aromatic hydrocarbon group or a fused aromatic hydrocarbon group selected from a group consisting of:
a phenyl group;
a naphthyl group;
a phenanthrenyl group;
a benzophenanthrenyl group;
a dibenzophenanthrenyl group;
a benzochrysenyl group;
a dibenzochrysenyl group;
a fluoranthenyl group;
a benzofluoranthenyl group;
a triphenylenyl group;
a benzotriphenylenyl group;
a dibenzotriphenylenyl group;
a picenyl group;
a benzopicenyl group;
a dibenzopicenyl group;
a phenalenyl group; and
a diazaphenanthrenyl group, and
when each of Ar¹ and Ar² is a phenyl group, (Ar³)ₙ-Ar⁴ is not structured as represented by a formula (8) below.

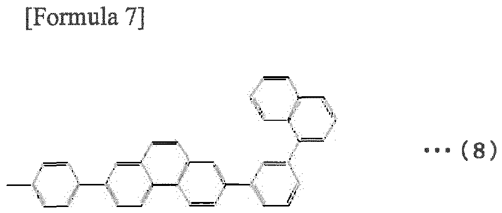

15. The monoamine derivative according to claim 12, wherein
each of Ar¹ and Ar² is a phenyl group or a naphthyl group, and
Ar⁴ is any one of a benzochrysenyl group, triphenylenyl group and phenanthrenyl group.

16. The monoamine derivative according to claim 13, wherein
each of Ar¹ and Ar² is a phenyl group or a naphthyl group,
Ar³ is a phenylene group or a naphthylene group, and
Ar⁴ is any one of a benzochrysenyl group, triphenylenyl group and phenanthrenyl group.

17. The monoamine derivative according to claim 14, wherein
each of Ar¹ and Ar² is a phenyl group or a naphthyl group,
Ar³ is a phenylene group or a naphthylene group, and
Ar⁴ is any one of a benzochrysenyl group, triphenylenyl group and phenanthrenyl group.

18. The monoamine derivative according to any one of claims 7 to 17 that is usable as a phosphorescent host material in an organic electroluminescence device.

19. An organic electroluminescence device comprising:
a cathode;
an anode; and
a single-layered or multilayered organic thin-film layer provided between the cathode and the anode, wherein
at least one layer of the organic thin-film layer comprises the monoamine derivative according to any one of claims 7 to 17.

20. The organic electroluminescence device according to claim 19, wherein the organic thin-film layer comprising the monoamine derivative further comprises at least one phosphorescent material.

21. The organic electroluminescence device according to claim 19, wherein the at least one layer of the organic thin-film layer is an emitting layer, and
at least one layer of the emitting layer comprises the monoamine derivative and at least one phosphorescent material.

22. The organic electroluminescence device according to claim 21, wherein the emitting layer comprises:
a first host material;
a second host material; and
a phosphorescent dopant material,
the second host material comprises the monoamine derivative, and
the phosphorescent dopant material is the at least one phosphorescent material.

23. The organic electroluminescence device according to claim 22, wherein the first host material is a fused aromatic compound represented by any one of formulae (11) and (12) below,
Ar^{a}-Ar^{b}-Ar^{c} (11)
Ar^{d}-Ar^{e}-Ar^{f}-Ar^{g} (12)
where Ar^{a}, Ar^{b}, Ar^{c}, Ar^{d}, Ar^{e}, Ar^{f} and Ar^{g} each independently represents a group derived from any one of skeletons below:
a substituted or unsubstituted benzene skeleton;
a substituted or unsubstituted naphthalene skeleton; and
a substituted or unsubstituted fused aromatic hydrocarbon skeleton having 3 or more rings.

24. The organic electroluminescence device according to claim 23, wherein the fused aromatic hydrocarbon skeleton having 3 or more rings for the first host material is any one of skeletons represented by formulae (13) to (16) below, 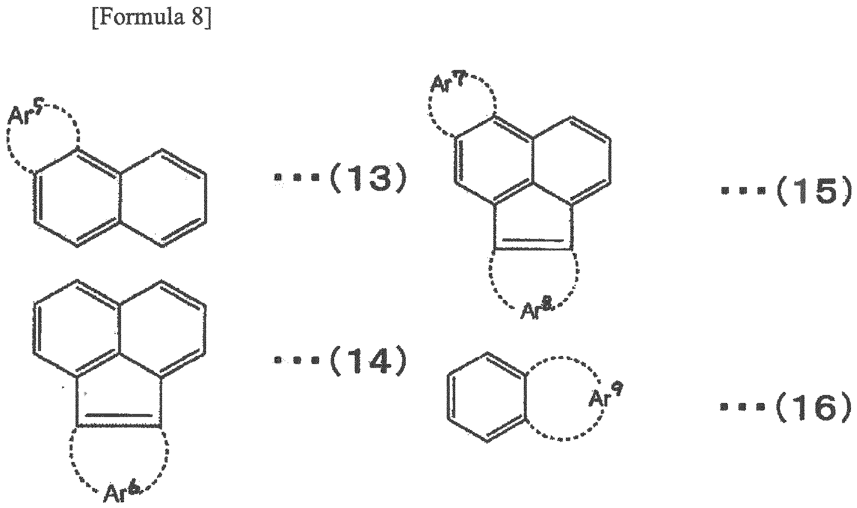 where each of Ar⁵, Ar⁶, Ar⁷, Ar⁸ and Ar⁹ has a substituted or unsubstituted cyclic structure having 4 to 20 ring carbon atoms and is combined with a ring adjacent thereto to form a fused ring.

25. The organic electroluminescence device according to claim 24, wherein the fused aromatic hydrocarbon skeleton having 3 or more rings for the first host material is any of skeletons below:
a phenanthrene skeleton;
a benzophenanthrene skeleton;
a dibenzophenanthrene skeleton;
a chrysene skeleton;
a benzochrysene skeleton;
a dibenzochrysene skeleton;
a fluoranthene skeleton;
a benzofluoranthene skeleton;
a triphenylene skeleton;
a benzotriphenylene skeleton;
a dibenzotriphenylene skeleton;
a picene skeleton;
a benzopicene skeleton; and
a dibenzopicene skeleton.

26. The organic electroluminescence device according to any one of claims 20 to 25, wherein
the phosphorescent material comprises a metal complex, and
the metal complex comprises: a metal atom selected from a group consisting of Ir, Pt, Os, Au, Re and Ru; and a ligand.

27. The organic electroluminescence device according to claim 26, wherein the ligand has an ortho-metal bond with the metal atom of the metal complex.

28. The organic electroluminescence device according to any one of claims 20 to 27, wherein a maximum emission wavelength of the at least one phosphorescent material contained in the emitting layer is in a range of 520 nm to 720 nm.

29. A monoamine derivative represented by a formula (1B) below, 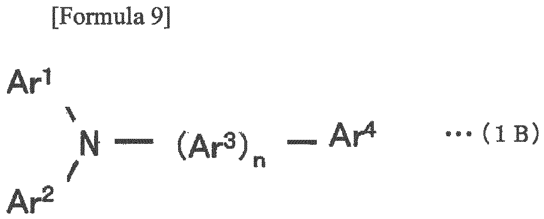 where each of Ar¹, Ar² and Ar⁴ is a substituted or unsubstituted aryl group or heteroaryl group,
Ar³ is a substituted or unsubstituted arylene group or heteroarylene group,
n is an integer of 0 to 5,
when n is 2 or more, Ar³ is allowed to be mutually the same or different,
when n is 1, Ar³ is a substituted or unsubstituted arylene group having 12 to 20 ring carbon atoms or heteroarylene group having 5 to 20 ring atoms, and
at least one of Ar¹, Ar² and Ar⁴ is a group derived from a fused aromatic hydrocarbon skeleton having 3 or more rings.
